(19) Europäisches Patentamt
European Patent Office
Office européen des brevets

(11) EP 0 525 360 B1

(12) **EUROPEAN PATENT SPECIFICATION**

(45) Date of publication and mention of the grant of the patent:
**25.03.1998 Bulletin 1998/13**

(51) Int. Cl.$^6$: **C07C 235/34**, C07C 237/20, A61K 31/165, C07D 317/58

(21) Application number: **92110157.2**

(22) Date of filing: **16.06.1992**

# (54) Novel phenylacetamide derivatives and processes for the preparation thereof

Neue Phenylacetamidderivate und Verfahren zu ihrer Herstellung

Nouveau dérivés de phénylacetamide et procédé pour leur préparation

(84) Designated Contracting States:
**CH DE FR GB IT LI**

(30) Priority: **30.07.1991 KR 1309491**
**30.07.1991 KR 1309591**
**30.07.1991 KR 1309691**
**06.11.1991 KR 1964191**

(43) Date of publication of application:
**03.02.1993 Bulletin 1993/05**

(73) Proprietor:
**KOREA RESEARCH INSTITUTE OF CHEMICAL TECHNOLOGY**
**Daejeon, 305-606 (KR)**

(72) Inventors:
- **Park, No Sang**
  **Daejeon (KR)**
- **Ha, Deok Chan**
  **Yuseong-ku, Daejeon (KR)**
- **Choi, Joong Kwon**
  **Yuseong-ku, Daejeon (KR)**
- **Kim, Hyun Sook**
  **Daejeon (KR)**
- **Hong, Mi Sook**
  **Daejeon (KR)**
- **Lim, Hee Jong**
  **Pusan (KR)**
- **Lee, Kwang Sook**
  **Chungcheongnam-do (KR)**

(74) Representative:
**Turi, Michael, Dipl.-Phys. et al**
**Samson & Partner**
**Widenmayerstrasse 5**
**80538 München (DE)**

(56) References cited:
**EP-A- 0 178 381** **EP-A- 0 282 127**
**EP-A- 0 401 903**

- **CHEMICAL ABSTRACTS, vol. 117, no. 5, 3 August 1992, Columbus, Ohio, US; abstract no. 48051d, NO SANG PARK ET AL. 'N-Aralkylated 4-(2-aminoethoxy)phenylacetamide derivatives as potent analgesic and antiinflammatory agents' page 879 ;column 1 ;**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 525 360 B1

Printed by Xerox (UK) Business Services
2.15.12/3.4

## Description

The present invention relates to novel phenylacetamide derivatives; and more specifically, relates to phenylacetamide derivatives which have powerful anti-inflammatory and analgesic activities, pharmaceutically acceptable salts thereof and processes for the preparation thereof.

Hitherto, it has been known that capsaicin of the following formula(A)

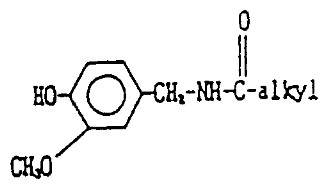

(A)

which is present in extract from fruit of a plant belonging to the genus Cavenne has an analgesic property. Such natural capsaicin(i. e., trans-8-methyl-N-vanillyl-6-nonenamide) and a synthetic capsaicin (N-vanillyl-nonanamide) have been described in US-A-4,313,958 as an analgesic. Also, similar results of chemical and pharmacological studies have been reported in various references [see, e.g., Yaksh, et al., Science, 206, pp 481-483(1979); and The Alkaloids, Vol. XXIII, pp 227-299(1984), Academic Press].

Described in EP-A-0,282,127 as having anti-inflammatory or analgesic activity are the aminoethoxybenzylamine derivatives of the following formula(B)

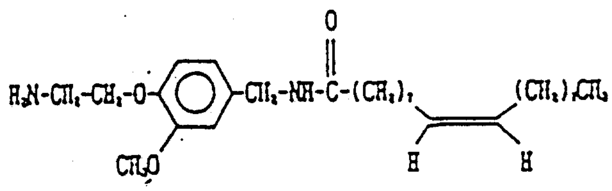

(B)

Further, US-A-4,424,205 (EP-A-0,089,710) discloses the analgesic and/or anti-irritant compounds of the following formula(C)

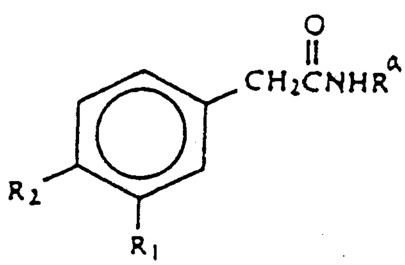

(C)

wherein $R^a$ is a linear or branched $C_3$-$C_{11}$ alkyl, alkynyl or aralkyl group, a linear or branched $C_3$-$C_{22}$ alkenyl group, or an unbranched or branched $C_6$-$C_{11}$ cycloalkyl or cycloalkenyl group; and one of $R_1$ and $R_2$ is OH, the other being OH or H.

GB-A-2,168,975 also provides aralkanamides having formula(D)

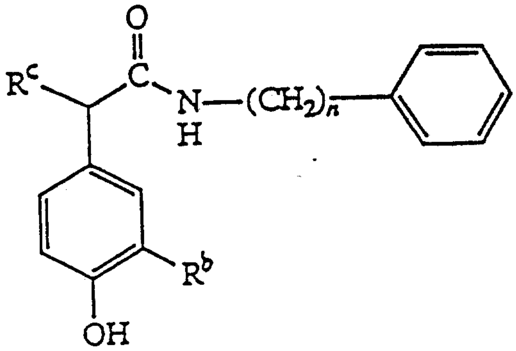

(D)

wherein $R^b$ represents H, OH, $OCH_3$ or $OCH_2CH_3$; $R^c$ represents H or $CH_3$; and n is an integer from 0 to 12.

EP-A-178 381 describes anti-asthma and anti-inflammatory phenylacetamides including compounds of formula

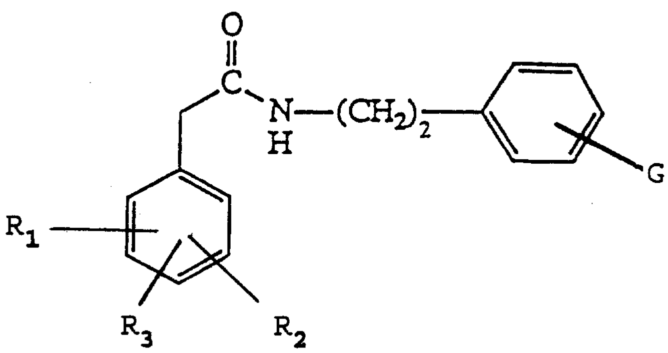

(E)

wherein $R_3$ is H or halogen;

$R_1$ and $R_2$ are independently OH, lower alkyl, lower alkoxy, aryloxy, heteroaryloxy, heteroaryl lower alkoxy, aryl, heteroaryl, aryl lower alkyl, aryl lower alkoxy, halogenated aryl lower alkoxy, lower alkenyl, lower alkynyl, lower alkenoxy, lower alkynoxy, halogen, trifluoromethyl, cyclopropylmethoxy or cyclohexenoxy; and

G is OH, lower alkyl, lower alkoxy, aryloxy, heteroaryl, aryl lower alkyl, aryl lower alkoxy, halogenated aryl lower alkoxy, lower alkenyl, lower alkynyl, lower alkenoxy, lower alkynoxy, halogen or trifluoromethyl.

US-A-5,045,565 to Gardner et al. and, similarly, EP-A-0 401 903 generically disclose β-aminoethoxy-substituted phenyl compounds, including a compound of formula(E):

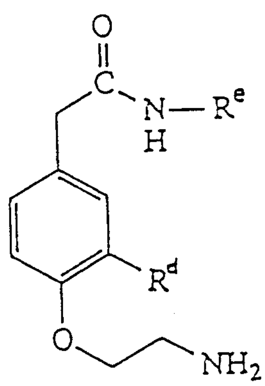

(F)

wherein,

$R^d$ is a hydrogen, or a hydroxy or methoxy group; and
$R^e$ is a $C_{1-24}$ alkyl group.

According to the detailed description of the Gardner patent the term "alkyl" is defined to include straight carbon chains optionally substituted with aryl, which is, in turn, defined to include a phenyl optionally substituted with a halogen, hydroxy, $C_1$-$C_{16}$ alkoxy, amino, nitro, cyano, phenyl, benzyl, benzyloxy, trifluoromethyl, formylamino, carboxylate or $C_1$-$C_6$ alkyl group.

Although the Gardner patent discloses alkyl substituted phenyl acetamide derivatives of the above formula(E), it fails to, e.g., describe N-n-alkyl substituted phenylacetamides with a phenyl group attached to the end of the n-alkyl group.

The foregoing prior art capsaicin derivatives can be characterized by their having a saturated or unsaturated alkyl group of 3 to 24 carbon atoms or a substituted or unsubstituted ω-phenyl alkyl group attached to the carbonyl group. These compounds are, however, generally known to have several side-effects: for example, strong irritation, reddening of skin and toxicity.

To ameliorate the afore-mentioned problems, therefore, the present inventors have sucessfully worked toward the development of novel phenylacetamide derivatives which have powerful analgesic and anti-inflammatory activities with little or much reduced level of irritation and toxicity. Specifically, the invention provides novel phenylacetamide derivatives of the following formula(I)

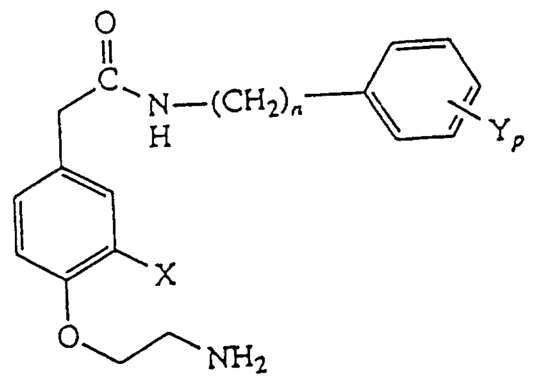

(I)

wherein:

X is a hydrogen, halogen, hydroxy, nitro, amino, $R^1$, $NR^1R^2$, $NHR^1$ or $OR^1$ wherein $R^1$ and $R^2$ are an optionally substituted $C_{1-8}$ alkyl, cycloalkyl or benzyl group, respectively;
Y, which may be the same or different when p is greater than 1, is a halogen, methylenedioxy, hydroxy, trifluoromethyl, $R^3$ or $OR^3$ wherein $R^3$ is an optionally substituted $C_{1-8}$ alkyl or benzyl group;
n is an integer from 1 to 6; and
p is an integer from 1 to 5.

In the context of the present invention and specification, unless otherwise specifically mentioned, the term "phenylacetamide derivatives" shall be taken to include pharmaceutically acceptable salts thereof.

In another aspect, the present invention provides processes for the preparation of these compounds and medicinal formulations containing them as an active ingredient.

Preferred compounds of the present invention include the compounds of formula(I) wherein X is a hydrogen, halogen, hydroxy, nitro, amino or $OR^1$; Y, which may be identical or different when p is greater than 1, is a halogen, trifluoromethyl or $R^3$; n is an integer from 2 to 5; p is an integer from 1 to 3; and $R^1$ and $R^3$ have the same meanings as defined above.

More preferred compounds of the present invention are the compounds of formula(I) wherein X is a halogen, hydroxy, nitro, amino or $OR^1$; Y, which may be identical or different when p is 2, is a halogen, trifluoromethyl or $R^3$; n is 3 or 4; p is 1 or 2; and $R^1$ and $R^3$ are an optionally substituted $C_{1-5}$ alkyl, respectively.

Pharmaceutically acceptable salts of the compounds having the formula(I) according to the present invention may be the salts of inorganic acids such as hydrochloric acid, hydrogen bromide, sodium hydrogen sulfate and carbonic acid, or of organic acids such as formic, acetic, oxalic, benzoic, citric, tartaric, gluconic, gentisic, fumaric and lactobionic

acids.

The present invention also includes within its scope pharmaceutical compositions comprising one or more of the phenylacetamide derivatives of formula(I) as active ingredients, in association with a possible pharmaceutically acceptable carrier. The term "(pharmaceutical) carrier", as used herein, means one or more compatible solid or liquid filler, diluents or encapsulating materials which are suitable for a human or animal administration. The compatible carriers, as used herein, are the components that do not cause any interactions which substantially reduce the efficacy of the pharmaceutical composition in an ordinary user environment. Possible pharmaceutical carriers must be of sufficiently low toxicity to make them suitable for administration to the subject of treatment.

Some examples of substances which can serve as the carrier are sugar, starch, cellulose and its derivatives, powdered tragacanth, malt, gelatin, talc, stearic acid, magnesium stearate, calcium sulfate, vegetable oils, polyols, alginic acid, pyrogen-free water, isotonic saline phosphate buffer solutions, cocoa buffer(suppository base), emulsifier as well as other non-toxic pharmaceutically compatible substances used in other pharmaceutical formulations. Wetting agents and lubricants such as sodium lauryl sulfate, as well as coloring agents, flavoring agents, excipients, tabletting agents, stabilizers, antioxidants and preservatives may also be present. Other compatible additives and ingredients such as pain killers, muscle relaxants may be included in the possible pharmaceutical carrier for use in the compositions of the present invention.

The proper pharmaceutical carriers of the present invention are basically determined by the administration route. The compounds of the present invention may be administered by injection, orally and topically. If the compound is to be injected, the preferred carrier is the sterile, physiological saline with pH 4. If the compound is to be applied topically, the carrier may preferably comprise those suited for use in creams, gels, tapes and the like. And the pharmaceutical carriers for oral administration may include those suited for tablets and capsules.

The carrier may comprise, in total, from about 0.1% to about 99.99999 % by weight of the pharmaceutical compositions of the present invention, mainly from about 50% to about 99. 9999%.

Total single dosages of the compounds of the present invention are generally from about 1μg to about 10g. Preferred single dosages are from about 1μg to about 3500mg; and more preferred are from about 1μg to 1000mg; and most preferred are from about 1μg to about 600mg.

Possible pharmaceutical carriers suitable for the preparation of unit dosage forms for oral administration and injection, and dosage forms for topical application are well-known in the art. Their selection may further depend on secondary considerations such as taste, cost, and/or shelf stability, which are not critical for the purposes of the present invention; and may be made without difficulty by a person skilled in the art.

The phenylacetamide derivatives having the formula(I) according to the present invention may be prepared in accordance with the procedure comprising the steps of: (i) reacting an amine compound of the formula(II) with a para-hydroxyphenylacetic acid of the formula(III) to provide a compound of the formula(IV); (ii) reacting the compound(IV) with 1,2-dibromoethane to provide a compound of the formula(V); (iii) reacting the compound(V) with sodium azide($NaN_3$) to provide a compound of the formula(VI); and then (iv) converting the compound(VI) to the compound of the formula(I), as further described below:

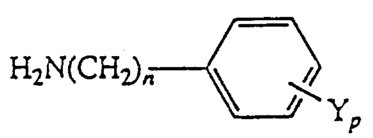

(II)

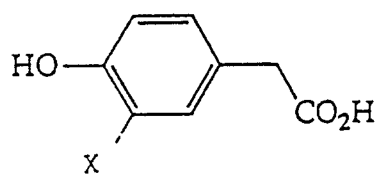

(III)

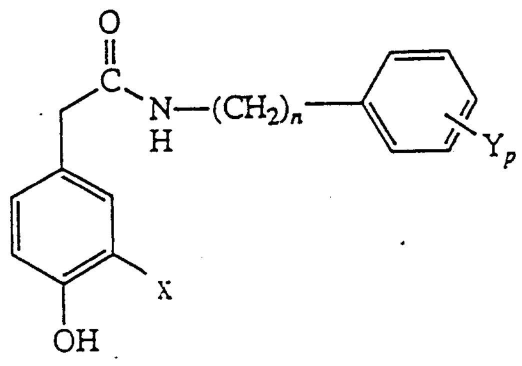

(IV)

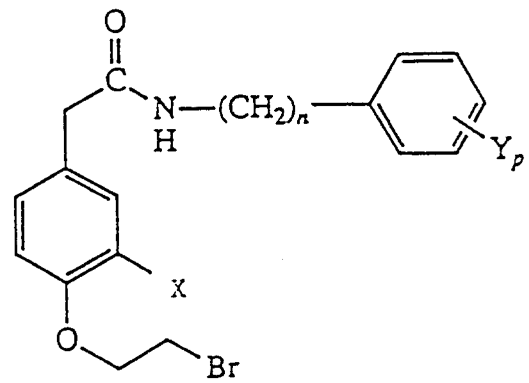

(V)

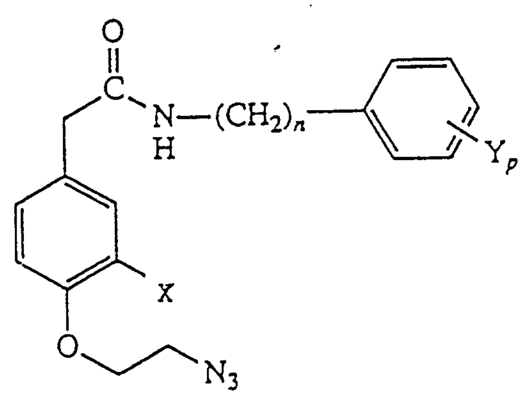

(VI)

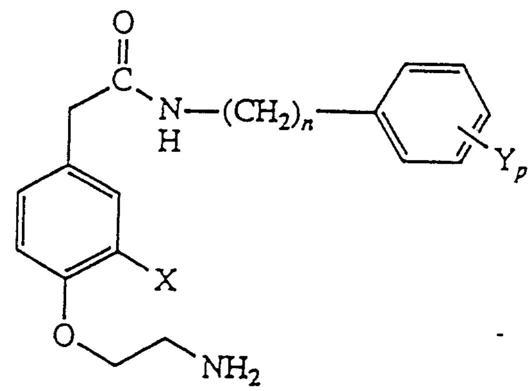

(I)

In the above process, step(i) may be conducted by heating the reactants at a temperature ranging from 130°C to 160°C for 2 to 5 hours in the presence of a catalyst such as 0.3 nm, 0.4 nm or 0.5 nm (3Å , 4Å or 5Å), and preferably 0.4 nm (4Å), powdered molecular sieve without any solvent.

Step(ii) may be conducted in the presence of a base such as sodium hydride(NaH) in a solvent, e.g., tetrahydrofuran, at the boiling point of the solvent.

Step(iii) of the process may be carried out in the presence of a catalyst such as n-tetrabutyl ammonium bromide(n - $Bu_4NBr$) in a solvent, e.g., benzene, at the boiling temperature of the solvent.

Finally, step(iv) may be carried out by reducing the product from step(iii) In the presence of palladium-on-carbon(Pd-C) as a catalyst under an elevated hydrogen pressure or by reacting the product of step(iii) with triphenylphosphine in the presence of water and the tetrahydrofuran solvent to obtain the compound of the formula(I) wherein X is nitro.

In step(i) above, the compound of the formula(IV) is obtained as an intermediate for preparing the final compound of the present invention; and it is found that the intermediate itself also has analgesic and anti-inflammatory effects.

An amine compound of the formula(II) used in preparing the compounds of the present invention as a starting material may be obtained from the compound of the formulae(VII) or from the compound of the formula(VIII)

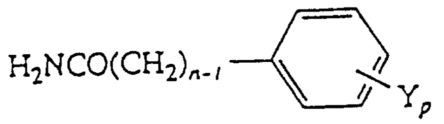

(VII)

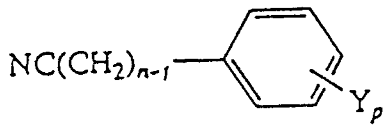

(VIII)

wherein Y, n and p have the same meanings as previously defined, by employing a conventional method such as a reduction method using, e.g., lithium aluminium hydride($LiAlH_4$), alane, etc., or a hydrogenation method using a metal such as palladium, Raney nickel as a catalyst. An acid compound of formula(III) is commercially available and also can be prepared using a partial protection method.

Specifically, representative examples of the phenyl acetamide derivatives having formula(I) of the present invention are as follows:

N-{3-(3,4-dimethylphenyl)propyl}-4-(2-aminoethoxy)-3-hydroxyphenylacetamide;
N-{2-(3,4-dimethylphenyl)ethyl}-4-(2-aminoethoxy)-3-hydroxyphenylacetamide;
N-{3-(3-methylphenyl)propyl}-4-(2-aminoethoxy)-3-hydroxyphenylacetamide;
N-{3-(4-methylphenyl)propyl}-4-(2-aminoethoxy)-3-hydroxyphenylacetamide;
N-{3-(4-chlorophenyl)propyl}-4-(2-aminoethoxy)-3-hydroxyphenylacetamide;
N-{3-(3,4-dichlorophenyl)propyl}-4-(2-aminoethoxy)-3-hydroxyphenylacetamide;
N-{3-(4-fluorophenyl)propyl}-4-(2-aminoethoxy)-3-hydroxyphenylacetamide;
N-{3-(3,4-methylenedioxyphenyl)propyl}-4-(2-aminoethoxy)-3-hydroxyphenylacetamide;
N-{3-(3-methoxyphenyl)propyl}-4-(2-aminoethoxy)-3-hydroxyphenylacetamide;
N-{3-(3-trifluoromethylphenyl)propyl}-4-(2-aminoethoxy)-3-hydroxyphenylacetamide;
N-{3-(3,5-dimethylphenyl)propyl}-4-(2-aminoethoxy)-3-hydroxyphenylacetamide;
N-{3-(3-ethylphenyl)propyl}-4-(2-aminoethoxy)-3-hydroxyphenylacetamide;
N-{4-(3,4-dimethylphenyl)butyl}-4-(2-aminoethoxy)-3-hydroxyphenylacetamide;
N-{3-(3,4-dimethylphenyl)propyl}-4-(2-aminoethoxy)-3-nitrophenylacetamide;
N-{2-(3,4-dimethylphenyl)ethyl}-4-(2-aminoethoxy)-3-nitrophenylacetamide;
N-{3-(3-methylphenyl)propyl}-4-(2-aminoethoxy)-3-nitrophenylacetamide;
N-{3-(4-methylphenyl)propyl}-4-(2-aminoethoxy)-3-nitrophenylacetamide;
N-{3-(4-chlorophenyl)propyl}-4-(2-aminoethoxy)-3-nitrophenylacetamide;
N-{3-(3,4-dichlorophenyl)propyl}-4-(2-aminoethoxy)-3-nitrophenylacetamide;
N-{3-(4-fluorophenyl)propyl}-4-(2-aminoethoxy)-3-nitrophenylacetamide;
N-{3-(3,4-methylenedioxyphenyl)propyl}-4-(2-aminoethoxy)-3-nitrophenylacetamide;
N-{3-(3-methoxyphenyl)propyl}-4-(2-aminoethoxy)-3-nitrophenylacetamide;
N-{3-(3-trifluoromethylphenyl)propyl}-4-(2-aminoethoxy)-3-nitrophenylacetamide;
N-{3-(3,5-dimethylphenyl)propyl}-4-(2-aminoethoxy)-3-nitrophenylacetamide;
N-{3-(3-ethylphenyl)propyl}-4-(2-aminoethoxy)-3-nitrophenylacetamide;
N-{4-(3,4-dimethylphenyl)butyl}-4-(2-aminoethoxy)-3-nitrophenylacetamide;
N-{3-(3,4-dimethylphenyl)propyl}-4-(2-aminoethoxy)-3-aminophenylacetamide;
N-{2-(3,4-dimethylphenyl)ethyl}-4-(2-aminoethoxy)-3-aminophenylacetamide;
N-{3-(3-methylphenyl)propyl}-4-(2-aminoethoxy)-3-aminophenylacetamide;
N-{3-(4-methylphenyl)propyl}-4-(2-aminoethoxy)-3-aminophenylacetamide;
N-{3-(4-chlorophenyl)propyl}-4-(2-aminoethoxy)-3-aminophenylacetamide;
N-{3-(3,4-dichlorophenyl)propyl}-4-(2-aminoethoxy)-3-aminophenylacetamide;
N-{3-(4-fluorophenyl)propyl}-4-(2-aminoethoxy)-3-aminophenylacetamide;
N-{3-(3,4-methylenedioxyphenyl)propyl}-4-(2-aminoethoxy)-3-aminophenylacetamide;
N-{3-(3-methoxyphenyl)propyl}-4-(2-aminoethoxy)-3-aminophenylacetamide;
N-{3-(3-trifluoromethylphenyl)propyl}-4-(2-aminoethoxy)-3-aminophenylacetamide;
N-{3-(3,5-dimethylphenyl)propyl}-4-(2-aminoethoxy)-3-aminophenylacetamide;
N-{3-(3-ethylphenyl)propyl}-4-(2-aminoethoxy)-3-aminophenylacetamide;
N-{4-(3,4-dimethylphenyl)butyl}-4-(2-aminoethoxy)-3-aminophenylacetamide;
N-{3-(4-chlorophenyl)propyl}-4-(2-aminoethoxy)phenylacetamide;

N-{3-(4-chlorophenyl)propyl}-4-(2-aminoethoxy)-3-fluorophenylacetamide;
N-{3-(4-chlorophenyl)propyl}-4-(2-aminoethoxy)-3-chlorophenylacetamide;
N-{3-(4-chlorophenyl)propyl}-4-(2-aminoethoxy)-3-methoxyphenylacetamide;
N-{3-(2,4-dichlorophenyl)propyl}-4-(2-aminoethoxy)-3-methoxyphenylacetamide;
N-{3-(3,4-dichlorophenyl)propyl}-4-(2-aminoethoxy)-3-methoxyphenylacetamide;
N-{3-(4-fluorophenyl)propyl}-4-(2-aminoethoxy)-3-methoxyphenylacetamide;
N-{3-(3,4-methylenedioxyphenyl)propyl}-4-(2-aminoethoxy)-3-methoxyphenylacetamide;
N-{3-(3-methoxyphenyl)propyl}-4-(2-aminoethoxy)-3-methoxyphenylacetamide;
N-{3-(3-benzyloxyphenyl)propyl}-4-(2-aminoethoxy)-3-methoxyphenylacetamide;
N-{3-(3,4-dimethoxyphenyl)propyl}-4-(2-aminoethoxy)-3-methoxyphenylacetamide; and
N-{3-(3-trifluoromethylphenyl)propyl}-4-(2-aminoethoxy)-3-methoxyphenylacetamide.

As will be amply demonstrated below, 4-(2-aminoethoxy) phenylacetamide derivatives of the formula(I) according to the present invention, which have various substitutents of ω-phenylalkyl group on the carbonyl radical, and pharmaceutically acceptable salts thereof, have superior anti-inflammatory and analgesic activities to any compounds known in the prior art.

The following Preparation Examples illustrate how the starting materials can be prepared. All NMR data are given in ppm.

Preparation Example 1: Synthesis of 3-(3,4-dimethylphenyl)propylamine

To a solution of 70.0mℓ of diethyl malonate dissolved in 400mℓ of dry ethanol was added 10.0g of metallic sodium. The reaction mixture was stirred for 30 minutes and cooled to 0°C; and 66.5g of 3,4-dimethylbenzyl chloride was added thereto. This reaction mixture was stirred for 1 hour at room temperature, heated at the boiling temperature for 4 hours and concentrated under reduced pressure to produce residues, which were dissolved in ethyl ether. This ethereal solution was washed with water. To the residues obtained after a further concentration of the organic phase were added 500mℓ of water and 170g of KOH; and this mixture was heated at the boiling temperature for 24 hours. This mixture was concentrated under reduced pressure until about a half of the amount remained, and 200mℓ of sulfuric acid was then slowly added thereto. The resultant solution was heated at the boiling temperature for 24 hours, extracted twice with 300mℓ of ethyl ether and evaporated under reduced pressure to obtain solids, which were recrystallized from boiling hexane to provide 42.1g of 3-(3,4-dimethylphenyl)propanoic acid as a white solid(yield 55%), having the characteristics of: m.p. 82°C; NMR($CDCl_3$, 300MHz)δ 2.22(s, 3H, $CH_3$), 2.24(s, 3H, $CH_3$), 2.66(t, J=8Hz, 2H, $ArCH_2$), 2.89(t, J=8Hz, 2H, $CH_2CO$), 6.93~7.70(m, 3H, ArH).

A mixture of 18.5g of 3-(3,4-dimethylphenyl)propanoic acid obtained above and 50mℓ of thionyl chloride was heated to reflux for 2 hours and concentrated under reduced pressure to give residues, which were dissolved in 100mℓ of ethyl ether. The ethereal solution was added to a mixture of 200mℓ of ethyl ether, 150mℓ of water and 50mℓ of 30% aqueous ammonia solution with stirring. The organic layer was separated and the remaining aqueous layer was extracted twice with 150mℓ of dichloromethane. The organic layers were combined and concentrated under reduced pressure to give residues, which were dissolved in 150mℓ of tetrahydrofuran. The solution was added to a mixture of 8.0g of $LiAlH_4$ and 200mℓ of tetrahydrofuran; and the resultant solution was heated at the boiling temperature for 6 hours. To this reaction mixture were slowly added 30mℓ of 30% NaOH aqueous solution and 20mℓ of water. The tetrahydrofuran layer was separated from the mixture and the remaining solids were dissolved in 300mℓ of water. The aqueous solution was extracted twice with 200mℓ of ethyl ether. The previously separated tetrahydrofuran solution and the ethyl ether solution were combined and then concentrated. The residues so obtained were distilled under reduced pressure to obtain 13.4g of 3-(3,4-dimethylphenyl)propylamine(yield 70%), having the characteristics of: b.p. 140-150°C/0.67 mbar (0.5mmHg); NMR($CDCl_3$, 300MHz)δ 1.32 (br s, 2H, $NH_2$), 1.74(quint, J=7Hz, 2H, $CH_2$), 2.23(s, 3H, $CH_3$), 2.24(s, 3H, $CH_3$), 2.59(t, J=7Hz, 2H, $ArCH_2$), 2.72(t, J=7Hz, 2H, $CH_2N$), 6.91-7.06(m, 3H, ArH).

Preparation Example 2: Synthesis of 3-(3-methylphenyl)propylamine

The procedures of Preparation Example 1 were repeated except that 3,4-dimethylbenzyl chloride was replaced with 3-methylbenzyl chloride to obtain the title compound (yield 82%), having the characteristics of: b.p. 150°C/0.40 mbar (0.3mmHg); NMR($CDCl_3$)δ 1.20(br s, 2H, $NH_2$), 1.76(m, 2H, $CH_2$), 2. 33(s, 3H, $ArCH_3$), 2.61(t, J=7Hz, 2H, $ArCH_2$), 2.72(t, J=7Hz, 2H, $CH_2NH_2$), 6.48-7.20(m, 4H, ArH).

Preparation Example 3: Synthesis of 3-(4-methylphenyl)propylamine

The procedures of Preparation Example 1 were repeated except that 3,4-dimethylbenzyl chloride was replaced

with 4-methylbenzyl chloride to obtain the title compound (yield 73%), having the characteristics of: b.p. 130°C/0.53 mbar (0.4mmHg); NMR($CDCl_3$)δ 1.55(br s, 2H, $NH_2$), 1.75(m, 2H, $CH_2$), 2.32(s, 3H, $ArCH_3$), 2.61(t, J=7Hz, 2H, $ArCH_2$), 2.72(t, J=7Hz, 2H, $CH_2NH_2$), 7.01(s, 4H, ArH).

Preparation Example 4: Synthesis of 3-(3,5-dimethylphenyl)propylamine

The procedures of Preparation Example 1 were repeated except that 3,4-dimethylbenzyl chloride was replaced with 3,5-dimethylbenzyl chloride (yield 71%), having the characteristics of: b.p. 150°C/0.40 mbar (0.3mmHg); NMR($CDCl_3$)δ 1.48(br s, 2H, $NH_2$), 1.74(quint, J=7Hz, 2H, $CH_2$), 2.21(s, 6H, $2ArCH_3$), 2.56(t, J=7Hz, 2H, $ArCH_2$), 2.70(t, J=7Hz, 2H, $CH_2NH_2$), 6.81(s, 3H, ArH).

Preparation Example 5: Synthesis of 2-(3,4-dimethylphenyl)ethylamine

To a solution of 6.8g of 90% sodium cyanide dissolved in 6.5mℓ of distilled water while being subjected to heating was added a solution of 15.5g of 3,4-dimethylbenzyl chloride in 20mℓ of dry ethanol; and the resultant mixture was heated at the boiling temperature for 10 hours and then cooled to room temperature. This solution was added to 50mℓ of distilled water and extracted twice with 150mℓ of ethyl ether. The organic solvent was removed to obtain 11.5g(yield 79%) of 3,4-dimethylbenzyl cyanide, which was dissolved in 50mℓ of dry tetrahydrofuran. The organic solution was slowly added to a dispersion of 6.0g of $LiALH_4$ suspended in 150mℓ of dry tetrahydrofuran; and the mixture was heated at the boiling point for 14 hours. This reaction mixture was cooled to room temperature; and 16mℓ of 1N NaOH and 8mℓ of distilled water were slowly added thereto. Thereafter, this reaction mixture was filtered through a Celite layer to obtain solids, which were dissolved in a mixture of 250mℓ of ethyl ether and 250mℓ of ethyl alcohol and filtered. After the filtrate was basified with 5N NaOH aqueous solution and extracted with dichloromethane(200mℓx2), the solvent was evaporated and distilled under reduced pressure to provide 5.6g(yield 48%) of the title compound.

Preparation Example 6: Synthesis of 3-(4-chlorophenyl)propylamine

A mixture of 6.75g of 4-chlorocinnamic acid and 0.3g of $CuSO_4 \cdot 5H_2O$ dispersed in 50mℓ of ethyl alcohol was stirred at room temperature for 10 minutes; and 23mℓ of 95% hydrazine was added thereto. This reactant was stirred for 18 hours with air bubbling at the speed of 1L/min. The reaction mixture was filtered through a Celite layer and 30mℓ of 1N NaOH was added thereto. The solution was washed twice with 30mℓ of dichloromethane, acidified with concentrated hydrochloric acid, extracted with 100mℓ of ethyl acetate four times and dried over magnesium sulfate. The solvent was evaporated to give 6.05g(yield 89%) of 3-(4-chlorophenyl)propanoic acid as a yellow solid.

5.90g of 3-(4-chlorophenyl) propanoic acid obtained above was dissolved in 6mℓ of thionyl chloride, heated to reflux for 2 hours and concentrated under reduced pressure. The residues thus obtained were dissolved in 100mℓ of dry ethyl ether and the solution was added dropwise to 150mℓ of $NH_4OH$ in an ice bath. The resultant solution was stirred at room temperature for 1 hour and extracted twice with 200mℓ of dichloromethane. The organic phase was washed with 100mℓ of a saturated sodium hydrogen carbonate aqueous solution and dried over magnesium sulfate. The solvent was evaporated to provide 4.62g(yield 78%) of 3-(4-chlorophenyl)propanamide.

4.62g of amide obtained above was dissolved in 50mℓ of dry tetrahydrofuran; and the solution was slowly added to a dispersion of 2.4g of $LiAlH_4$ in 150mℓ of dry tetrahydrofuran with reflux for 2 hours. The reaction mixture was cooled to room temperature, treated with 10mℓ of 1N NaOH and filtered through a Celite layer to give solids, which were dissolved in 150mℓ of distilled water and then filtered through a Celite layer. The filtrate so obtained was extracted with ethyl ether(100mℓ x 3). The combined organic layer was dried over magnesium sulfate, concentrated and distilled under reduced pressure to provide 3.12g(yield 73%) of the title compound as a colorless liquid: b.p. 140°C/4.50 mbar (3.4mmHg).

Preparation Example 7: Synthesis of 3-(4-fluorophenyl)propylamine

The procedures of Preparation Example 6 were repeated except that 4-chlorocinnamic acid was replaced with 4-fluorocinnamic acid to obtain the title compound, having the characteristics of: NMR ($CDCl_3$)δ 1.35(s, 2H, $NH_2$), 1.73(m, 2H, $CH_2$), 2.65(m, 4H, $2CH_2$), 6.71(m, 2H, ArH), 7.03(m, 2H, ArH).

Preparation Example 8: Synthesis of 3-(2,4-dichlorophenyl)propylamine

The procedures of Preparation Example 6 were repeated except that 4-chlorocinnamic acid was replaced with 2,4-dichlorocinnamic acid to obtain the title compound, having the characteristics of: b.p. 116°C/1.00 mbar (0.75mmHg); NMR($CDCl_3$)δ 1.13(s, 2H, $NH_2$), 1.81(m, 2H, $CH_2$), 2.78(m, 4H, $2CH_2$), 7.27(m, 3H, ArH)

Preparation Example 9: Synthesis of 3-(3,4-dichlorophenyl)propylamine

The procedures of Preparation Example 6 were repeated except that 4-chlorocinnamic acid was replaced with 3,4-dichlorocinnamic acid to obtain the title compound, having the characteristics of: NMR ($CDCl_3$)δ 1.45(s, 2H, $NH_2$), 1.72(quint, J=7.4Hz, 2H, $CH_2$), 2.60(t, J=7.8Hz,2H, $CH_2N$), 2.70(t, J=7.1Hz, 2H, $ArCH_2$), 7.00(dd, J=2.1, 8.1Hz, 1H, ArH), 7.26(d, J=2.0Hz, 1H, ArH), 7.31(d, J=8.2Hz, 1H, ArH).

Preparation Example 10: Synthesis of 3-(3,4-methylenedioxyphenyl)propylamine

To a solution of 10.0g of 3,4-methylenedioxycinnamic acid dissolved in 130mℓ of 10% NaOH and 80mℓ of distilled water was added 1.0g of 5% Pd-C. This mixture was reacted at 40 psi until the consumption of hydrogen gas was ceased and then filtered through a Celite layer. The filtrate was acidified with concentrated hydrochloric acid, allowed to be cooled and filtered to give solids. The obtained solids were washed twice with 100mℓ of cold water and dried to provide 9.8g(yield 97%) of 3-(3,4-methylenedioxyphenyl) propanoic acid, having the characteristics of: NMR($CDCl_3$, 200MHz) δ 2.62(t, J=7Hz, 2H, $CH_2$), 2.88(t, J=7Hz, 2H, $CH_2N$), 5.93(s, 2H, $CH_2O_2$), 6.63-6.76(s, 3H, ArH).

A mixture of 8.23g of the propanoic acid obtained above and 5.3mℓ of thionyl chloride was heated to reflux for 2 hours and concentrated under reduced pressure to give residues, which were dissolved in 100mℓ of dry ethyl ether. This solution was slowly added dropwise to 150mℓ of $NH_4OH$ solution in an ice bath. The resultant mixture was stirred at room temperature for 1 hour, extracted with ether(200mℓ x 3) and dried over magnesium sulfate; and the solvent was removed therefrom to provide 7.89g(yield 96%) of 3-(3,4-methylenedioxyphenyl)propanamide.

6.0g of the above amide was dissolved in 100mℓ of dry tetrahydrofuran, which was added dropwise to a suspension of 2.4g of $LiAlH_4$ in 150mℓ of dry tetrahydrofuran. The reaction mixture was refluxed for 2 hours and 10mℓ of 1N NaOH was added thereto. The mixture was filtered through a Celite layer to give solids, which were dissolved in 200mℓ of distilled water. This solution was extracted twice with 200mℓ of ethyl ether. The combined organic phase was dried over magnesium sulfate, concentrated and distilled under reduced pressure to provide 4.71g(yield 85%) of 3-(3,4-methylenedioxyphenyl) propylamine, having the characteristics of: NMR($CDCl_3$, 200MHz)δ 1.34 (br s, 2H, $NH_2$), 1.72(m, J=7Hz, 2H, $CH_2$), 2.57(t, J=7Hz, 2H, $CH_2$), 2. 71(t, J=7Hz, 2H, $CH_2N$), 5.91(s, 2H, $CH_2O_2$), 6.59-6.74(s, 3H, ArH).

Preparation Example 11: Synthesis of 3-(3-trifluoromethylphenyl)propylamine

The procedures described in Preparation Example 10 above were repeated except that 3,4-methylenedioxycinnamic acid was replaced with 3-trifluoromethylcinnamic acid to obtain the title compound, having the characteristics of: NMR($CDCl_3$)δ 1.40-2.10(m, 4H, $CH_2$, $NH_2$), 2.40-2.90(m, 4H, $2CH_2$), 7.30(s, 4H, ArH).

Preparation Example 12: Synthesis of 3-(3-benzyloxyphenyl)propylamine

The procedures described in Preparation Example 10 above were repeated except that 3,4-methylenedioxycinnamic acid was replaced with 3-benzyloxycinnamic acid to obtain the title compound.

Preparation Example 13: Synthesis of 3-(3-methoxyphenyl)propylamine

The procedures described in Preparation Example 10 above were repeated except that 3,4-methylenedioxycinnamic acid was replaced with 3-methoxycinnamic acid to obtain the title compound, having the characteristics of: NMR($CDCl_3$)δ 1.24(s, 2H, $CH_2$), 1.72 (m, 2H, $CH_2$), 2.61(m, 4H, $2CH_2$), 3.68(s, 3H, $OCH_3$), 6.71(m, 3H, ArH), 7.18(m, 1H, ArH).

Preparation Example 14: Synthesis of 3-(3,4-dimethoxyphenyl)propylamine

The procedures described in Preparation Example 10 above were repeated except that 3,4-methylenedioxycinnamic acid was replaced with 3,4-dimethoxycinnamic acid to obtain the title compound (yield 73%).

Preparation Example 15: Synthesis of 3-(4-methoxyphenyl)propylamine

The procedures described in Preparation Example 10 above were repeated except that 3,4-methylenedioxycinnamic acid was replaced with 4-methoxycinnamic acid to obtain the title compound(yield 94%), having the characteristics of: NMR($CDCl_3$)δ 1.21(s, 2H, $NH_2$), 1.71 (m, 2H, $CH_2$), 2.57(t, J=7Hz, 2H, $CH_2$), 2.69(t, J=7Hz, 2H, $CH_2$), 3.75(s, 3H, $OCH_3$), 6.81-7.10(m, 4H, ArH).

Preparation Example 16: Synthesis of 4-(3-methylphenyl)butylamine

A mixture of 14.0g of 3-methylbenzyl chloride and 50.0g of ethyl acrylate dissolved in 300mℓ of dry toluene was heated to 110°C. Separatively, 3.0g of 2,2'-azobisisobutyronitrile(AIBN) and 32.0g of tri-n-butyltin hydride were dissolved in 200mℓ of dry toluene, which was added to the above mixture over a period of 2 hours. This reaction mixture was further heated at the boiling temperature for 2 hours and then cooled to the ambient temperature. The residues obtained after removing the solvent from the mixture were purified by silica gel column chromatography to provide ethyl 4-(3-methylphenyl)butanoate.

Hereto was introduced 100mℓ of 10% KOH aqueous solution; and the mixture was heated at the boiling temperature for 12 hours. The organic layer was washed with 50mℓ of ethyl ether, and the aqueous layer was acidified with concentrated hydrochloric acid and extracted with ethyl ether(100mℓ x 3). The organic solvent was removed under a reduced pressure to provide 9.6g(yield 55%) of 4-(3-methylphenyl)butanoic acid in the form of an emulsion.

A mixture of 3.3g of 4-(3-methylphenyl)butanoic acid obtained above and 10mℓ of thionyl chloride was heated at the boiling point for 2 hours and concentrated under reduced pressure to obtain residues, which were dissolved in 50mℓ of ethyl ether. This solution was added to a mixture of 60mℓ of ethyl ether, 300mℓ of water and 20mℓ of 30% aqueous ammonia solution with stirring. The organic layer was extracted with dichloromethane(50mℓ x 2). The combined organic layer was evaporated under reduced pressure to give residues, which were dissolved in 40mℓ of tetrahydrofuran. This solution was mixed with a mixture of 0.94g of $LiAlH_4$ and 60mℓ of tetrahydrofuran, which reaction mixture was then heated at the boiling point for 5 hours. To the above mixture was slowly added 10mℓ of 10% NaOH solution. The tetrahydrofuran layer was separated; and the residues were dissolved in 100mℓ of water. This aqueous solution was extracted with ethyl ether(50mℓ x 2). The ethyl ether layer and the separated tetrahydrofuran layer were combined and concentrated under reduced pressure. The residues so obtained were distilled under reduced pressure to provide 1.48g(yield 79%) of the title compound, having the characteristics of: b.p. 145-150°C/0.47 mbar (0.35mmHg); NMR($CDCl_3$)δ 1.49 (m, 2H, $NH_2$), 1.59-1.67(m, 4H, $2CH_2$), 2.33(s, 3H, $ArCH_3$), 2.59 (t, J=7Hz, 2H, $ArCH_2$), 2.70(t, J=7Hz, 2H, $NHCH_2$), 6.97-7.20(m, 4H, ArH).

Preparation Example 17: Synthesis of 4-(3,4-dimethylphenyl)butylamine

The title compound was prepared according to the procedures described in Preparation Example 16 above except for using 3,5-dimethylbenzyl chloride instead of 3-methylbenzyl chloride.

Preparation Example 18: Synthesis of 3-benzyloxy-4-hydroxyphenylacetic acid

To a solution of 5.79g of 3,4-dihydroxyphenyl acetic acid ethyl ester dissolved in dry acetone were added 4.48g of potassium carbonate and 4.15mℓ of benzyl bromide. The mixture was heated to reflux until the starting materials were exhausted; and then the solvent was distilled off under reduced pressure. To the residues thus obtained was added 50mℓ of water; and the mixture was acidified with concentrated hydrochloric acid and extracted with dichloromethane. The organic layer was dried over magnesium sulfate and evaporated under reduced pressure to obtain residues, which were purified by chromatoghraphy to provide 3.80g (yield 45%) of 3-benzyloxy-4-hydroxyphenylacetic acid ethyl ester, having the characteristics of: NMR($CDCl_3$, 200MHz)δ 1.24(t, J=7Hz, 3H, $CH_3$), 3.45(s, 2H, $CH_2CO$), 4.12(q, J=7Hz, 2H, $OCH_2$), 5.05(s, 2H, $CH_2Ph$), 6.77-7.35(m, 6H, ArH).

5.35g of the ester obtained above was added to a mixture of 50mℓ of water and 1.49g of sodium hydroxide. The resultant solution was refluxed for 2.5 hours, acidified with concentrated hydrochloric acid and evaporated under reduced pressure to give solids, which were purified by Soxhlet extraction apparatus to provide 4.27g(yield 89%) of the desired compound as a yellow solid, having the characteristics of: m.p. 130-133°C; NMR($CDCl_3$, 200MHz)δ 3.45(s, 2H, $CH_2Ar$), 5.09 (s, 2H, $OCH_2$), 6.75-7.40(m, 6H, ArH).

The following Examples illustrate how some of the compounds of formula(I) can be prepared.

Example 1: Synthesis of N-{3-(3,4-dimethylphenyl)propyl}-4-(2-aminoethoxy)-3-methoxyphenylacetamide

Step 1) Synthesis of N-{3-(3,4-dimethylphenyl)propyl}-4-hydroxy-3-methoxyphenylacetamide

A mixture of 1.39g of 3-(3,4-dimethylphenyl)propylamine obtained in Preparation Example 1, 1.50g of 4-hydroxy-3-methoxyphenylacetic acid and 0.60g of powdered 0.4 nm (4Å) molecular sieve was stirred for 4 hours at a temperature within the range of 150-160°C and dissolved in 10mℓ of dichloromethane. The resultant mixture was purified by column chromatography to provide 2.43g(yield 90%) of the title compound, having the characteristics of: NMR($CDCl_3$)δ 1.72(m, 2H, $CH_2$), 2.21(s, 6H, $2ArCH_3$), 2.49(t, J=7Hz, 2H, $ArCH_2$), 3.22(q, J=7Hz, 2H, $ArCH_2$), 3.47(s, 2H, $CH_2CO$), 3.87(s, 3H, $OCH_3$), 5.44 (s, 1H, NH), 5.78(s, 1H, OH), 6.68-7.04(m, 6H, ArH).

Step 2) Synthesis of N-{3-(3,4-dimethylphenyl)propyl}-4-(2-bromoethoxy)-3-methoxyphenylacetamide

The amide obtained in step 1 (1.80 g) was dissolved in a mixed solvent of 60mℓ of tetrahydrofuran and 10mℓ of 2,2-dibromoethane; and then 2.2g of 50% NaH was added thereto. The reaction mixture was heated at the boiling temperature for 12 hours and charged into 200mℓ of water. This was extracted with dichloromethane(150mℓ x 2) and evaporated under reduced pressure to remove the solvent. The residue so obtained was purified by column chromatography and recrystallized from dichloromethane-hexane to provide 1.53g(yield 63%) of the title compound, having the characteristics of: m.p.105°C; NMR($CDCl_3$)δ 1.73 (quint, J=7Hz, 2H, $CH_2$), 2.21(s, 6H, $2ArCH_2$), 2.50(t, J=7Hz, 2H, $ArCH_2$), 3.22(q, J=7Hz, 2H, $NCH_2$), 3.48(s, 2H, $CH_2CO$), 3.66(t, J=6Hz, 2H, $CH_2Br$), 3.86(s, 3H, $OCH_3$), 4.32(t, J=6Hz, 2H, $OCH_2$), 5.38(br s, 1H, NH), 6.73-7.05(m, 6H, ArH).

Step 3) Synthesis of N-{3-(3,4-dimethylphenyl)propyl}-4-(2-azidoethoxy)-3-methoxyphenylacetamide

To a solution containing 1.16g of 4-(2-bromoethoxy)phenylacetamide compound obtained in step 2 dissolved in 20mℓ of benzene were added 0.90g of $NaN_3$ and 0.17g of $n\text{-}Bu_4NBr$. The reaction mixture was heated at the boiling temperature for 24 hours, diluted with 100mℓ of dichloromethane and washed with 100mℓ of water. The solvent was removed under reduced pressure to give residues, which were purified by column chromatography and recrystallized from dichloromethane-hexane to provide 1.03g(yield 97%) of the title compound, having the characteristics of: m.p. 105°C; NMR($CDCl_3$)δ 1.73 (quint, J=7Hz, 2H, $CH_2$), 2.22(s, 6H, $2ArCH_3$), 2.50(t, J=7Hz, 2H, $ArCH_2$), 3.23(q, J=7Hz, 2H, $NCH_2$), 3.49(s, 2H, $CH_2CO$), 3.64(t, J=5Hz, 2H, $OCH_2$), 3.86(s, 3H, $OCH_3$), 4.18(t, J=5Hz, 2H, $CH_2N_3$), 5.40(br s, 1H, NH), 6.73-7.06(m, 6H, ArH).

Step 4) Synthesis of N-{3-(3,4-dimethylphenyl)propyl}-4-(2-aminoethoxy)-3-methoxyphenylacetamide

To a solution containing 1.00g of 4-(2-azidoethoxy)phenylacetamide compound obtained in step 3 dissolved in 100mℓ of ethyl acetate was added 0.30g of 10% Pd-C. This reaction mixture was reacted for 5 hours at the hydrogen pressure of about 40 psi, filtered through a Celite layer to remove Pd-C and evaporated under reduced pressure to remove the solvent. The residues so obtained were recrystallized from dichloromethane-hexane to provide 0.88g(yield 94%) of the desired compound, having the characteristics of: m.p. 125°C; NMR($CDCl_3$)δ 1.72(m, 4H, $CH_2$, $NH_2$), 2.21(s, 6H, $2ArCH_3$), 2.49(t, J=7Hz, 2H, $ArCH_2$), 3.11(q, J=5Hz, 2H, $OCH_2$), 3.22(q, J=7Hz, 2H, $NCH_2$), 3.49(s, 2H, $CH_2CO$), 3.85(s, 3H, $OCH_3$), 4.04(t, J=5Hz, 2H, $CH_2N$), 5.42(br s, 1H, NH), 6.71-7.03(m, 6H, ArH).

Examples 2 to 32

In accordance with the procedures described in Example 1, further compounds were prepared. Their particulars are as shown in Table 1.

Example 33: Synthesis of N-{3-(3,4-dimethylphenyl)propyl}-4-(2-aminoethoxy)-3-hydroxyphenylacetamide

Step 1) Synthesis of N-{3-(3,4-dimethylphenyl)propyl}-3-benzyloxy-4-hydroxyphenylacetamide

The procedures described in step 1 of Example 1 were repeated except that 4-hydroxy-3-methoxyphenylacetic acid was replaced with 3-benzyloxy-4-hydroxyphenylacetic acid to obtain the title compound (yield 87%), having the characteristics of: m.p. 63-66°C; NMR($CDCl_3$, 200MHz)δ 1.70(m, J=7Hz, 2H, $CH_2$), 2.19(s, 6H, $2CH_3$), 2.45(t, J=7Hz, 2H, $CH_2$), 3.18(q, J=5.1Hz, 2H, $CH_2N$), 3.41(s, 2H, $CH_2Ar$), 5.02(s, 2H, $CH_2$), 5.63(b, 1H, NH), 6.68-7.35(m, 6H, ArH).

Step 2) Synthesis of N-{3-(3,4-dimethylphenyl)propyl}-3-benzyloxy-4-(2-bromoethoxy)phenylacetamide

The procedures described in step 2 of Example 1 were repeated to obtain the title compound (yield 85%) having the characteristics of: m.p. 73-75°C; NMR($CDCl_3$, 200MHz)δ 1.71(m, J=7Hz, 2H, $CH_2$), 2.22(s, 6H, $2CH_3$), 2.50(t, J=7Hz, 2H, $CH_2$), 3.19(q, J=6.7Hz, 2H, $CH_2NH$), 3.45(s, 2H, $CH_2CO$), 3.65(t, J=6.4Hz, 2H, $CH_2Br$), 4.35(t, J=6.4Hz, 2H, $CH_2O$), 5.14(s, 2H, $OCH_2Ph$), 5.25(b, 1H, NH), 6.73-7.47(m, 6H, ArH).

Step 3) Synthesis of N-{3-(3,4-dimethylphenyl)propyl}-3-benzyloxy-4-(2-azidoethoxy)phenylacetamide

The procedures described in step 3 of Example 1 were repeated to obtain the title compound (yield 84%), having the characteristics of: m.p. 105-106°C; NMR($CDCl_3$, 200MHz)δ 1.64(m, J=6.5Hz, 2H, $CH_2$), 2.21(s, 6H, $2CH_3$), 2.49(t, J=8Hz, 2H, $CH_2$), 3.20(q, J=6.8Hz, 2H, $CH_2N$), 3.45(s, 2H, $CH_2Ar$), 3.61(t, J=5Hz, 2H, $CH_2N_3$), 4.20(t, J=5.1Hz, 2H,

$OCH_2$), 5.11(s, 2H, $OCH_2Ph$), 5.30(b, 1H, NH), 6.74-7.45(m, 6H, ArH).

Step 4) Synthesis of N-{3-(3,4-dimethylphenyl)propyl}-4-(2-aminoethoxy)-3-hydroxyphenylacetamide

The procedures described in step 4 of Example 1 were repeated to obtain the title compound(yield 87%), having the characteristics of: m.p. 144-147°C; NMR($CDCl_3$, 200MHz)δ 1.65(m, J=6.6Hz, 2H, $CH_2$), 2.10(s, 6H, $2CH_3$), 2.40(t, J=8Hz, 2H, $CH_2$), 2.95(t, J=5Hz, 2H, $CH_2N$), 3.18(m, J=6.7Hz, 2H, $CH_2NCO$), 3.35(s, 2H, $CH_2Ar$), 3.92(t, J=5Hz, 2H, $OCH_2$), 5.85(b, 1H, NH), 6.68-7.31(m, 6H, ArH).

Examples 34 to 36

In accordance with the procedures described in Example 36, various compounds were prepared. The results are shown in Table 1.

Example 37: Synthesis of N-{3-(3,4-dimethylphenyl)propyl}-4-(2-aminoethoxy)-3-aminophenylacetamide

Step 1) Synthesis of N-{3-(3,4-dimethylphenyl)propyl}-4-hydroxy-3-nitrophenylacetamide

A mixture of 7.0g of 4-hydroxy-3-nitrophenylacetic acid and 8.11g of $(COCl)_2$ suspended in 15mℓ of dichloromethane was heated to reflux for 2 hours and concentrated under reduced pressure to give residues, which were dissolved in dichloromethane. Hereto 4.14g of triethylamine and 6.37g of 3-(3,4-dimethylphenyl)propylamine were added, and the mixture was stirred at room temperature for 5 hours. The solvent was evaporated off and the obtained residues were purified by chromatography and recrystallized from dichloromethanehexane to provide 10.98g(yield 90%) of the title compound, having the characteristics of: m.p. 103°C; NMR ($CDCl_3$, 200MHz) δ 1.77 (quint, J=7Hz, 2H, $CH_2$), 2.23 (s, 6H, $CH_3$), 2.55 (t, J=8Hz, 2H, $CH_2Ar$), 3.29 (q, J=6.7Hz, 2H, $CH_2NH$), 3.46 (s, 2H, $CH_2CO$), 5.40 (br s, 1H, NH), 6.86-7.75 (m, 6H, ArH).

Step 2) Synthesis of N-{3-(3,4-dimethylphenyl)propyl}-4-(2-bromoethoxy)-3-nitrophenylacetamide

The procedures described in step 2 of Example 1 were repeated to obtain the title compound (yield 94%), having the characteristics of: m.p. 93°C; NMR ($CDCl_3$, 300MHz) δ 1.80 (quint, J=7Hz, 2H, $CH_2$), 2.23 (s, 6H, $CH_3$), 2.57 (t, J=8Hz, 2H, $CH_2Ar$), 3.25 (q, J=6.8Hz, 2H, $CH_2NH$), 3.46 (s, 2H, $CH_2CO$), 3.45 (t, J=6.5Hz, 2H, $CH_2Br$), 3.68 (t, J=6.5Hz, 2H, $CH_2O$), 5.38 (br s, 1H, NH), 6.86-7.72 (m, 6H, ArH).

Step 3) Synthesis of N-{3-(3,4-dimethylphenyl)propyl}-4-(2-azidoethoxy)-3-nitrophenylacetamide

The procedures described in step 3 of Example 1 were repeated to obtain the title compound(yield 97%), having the characteristics of: m.p. 89°C; NMR ($CDCl_3$, 300MHz) δ 1.80 (quint, J=7Hz, 2H, $CH_2$), 2.27 (s, 6H, $CH_3$), 2.57 (t, J=8Hz, 2H, $CH_2Ar$), 3.27 (q, J=6.8Hz, 2H, $CH_2NH$), 3.48 (s, 2H, $CH_2CO$), 3.69 (t, J=4.9Hz, 2H, $CH_2N_3$), 4.27 (t, J=4.9Hz, 2H, $CH_2O$), 5.40 (br s, 1H, NH), 6.88-7.75 (m, 6H, ArH).

Step 4) Synthesis of N-{3-(3,4-dimethylphenyl)propyl}-4-(2-aminoethoxy)-3-aminophenylacetamide

The procedures described in step 4 of Example 1 were repeated to obtain the title compound(yield 94%), having the characteristics of: m.p. 98°C; NMR (DMSO-$d_6$, 200MHz) δ 1.65 (quint, J=7Hz, 2H, $CH_2$), 2.18 (s, 6H, $CH_3$), 2.42-2.54 (m, 2H, $CH_2$), 2.95-3.07 (m, 2H, $CH_2$), 3.28 (s, 2H, $CH_2CO$), 3.30-3.45 (m, 2H, $CH_2$), 3.79-3.86 (m, 2H, $CH_2$), 4.78-4.85 (m, 2H, $ArNH_2$), 6.35-7.05 (m, 6H, ArH), 7.85-7.91 (br s, 1H, NH).

Examples 38-42

Further compounds of the formula(I) were prepared in accordance with the procedures described in Example 37. The results are shown in Table 1.

Example 43: Synthesis of N-{3-(3,4-dimethylphenyl)propyl}-4-(2-aminoethoxy)-3-nitrophenylacetamide

To a solution of 685mg of azide compound obtained in step 3 of Example 37 dissolved in 6mℓ of tetrahydrofuran was added 437mg of triphenylphosphine. This reaction mixture was stirred at room temperature for 2 hours and 0.1mℓ of water was added thereto. This mixture was stirred at room temperature for 4 hours and evaporated under reduced

pressure to remove the solvent. The residue was purified by chromatoghaphy and recrystallized from dichloromethane-hexane to provide 525mg(yield 82%) of the desired compound as a yellow precipitate, having the characteristics of: m.p. 98°C; NMR ($CDCl_3$, 200MHz)δ 1.77(m, J=7Hz, 2H, $CCH_2C$), 2.23(s, 6H, $CH_3$), 2.55(t, J=8Hz, 2H, $CH_2Ar$), 3.15(t, J=5.0Hz, 2H, $CH_2NH_2$), 3.29 (q, J=6.7Hz, 2H, $NHCH_2$), 3.46(s, 2H, $CH_2CO$), 4.15(t, J=5.0Hz, 2H, $OCH_2$), 5.40(br s, 1H, NH), 6.86-7.75(m, 6H, ArH).

Examples 44-46

Further compounds were prepared in accordance with the procedures described in Example 43. The results are shown in Table 1.

The following Use Examples illustrate how some of the pharmaceutical compositions of the present invention can be prepared.

Use Example 1

Tablets were prepared by using conventional methods, e.g., mixing and direct compression, and formulated as follows:

| Ingredients | mg per tablet |
|---|---|
| The Compound of Example 1 | 10 |
| Compressible sugar(Di-pac) | 400 |
| Sodium starch glycolate | 35 |
| Silica Gel(Syloid 244) | 15 |

One tablet was administered orally to a patient(male; age: 42; weight: 67kg) in need of analgesia two times daily to successfully provide the effect of general analgesia.

Use Example 2

Capsules for oral administration were prepared by combining the following ingredients:

| Ingredients | Amount |
|---|---|
| The Compound of Example 8 | 20mg |
| Sesame oil | 100mℓ |

The compound of Example 5 was dissolved in sesame oil with the aid of sonication and was packaged in soft gelatin capsules using the common methods known in the art. Two of the resulting capsules, each containing 27mg of the composition, were administered to a 63Kg male(age: 35) in need of treatment, producing the effects of analgesia and reducing inflammation.

Use Example 3

Syrup for oral administration was prepared by combining the following ingredients:

| Ingredients | Amount |
|---|---|
| The Compound of Example 1 | 250g |
| Benzoic Acid Solution | 20mℓ |
| Compound Tartrative Solution | 10mℓ |
| Water for preparations | 20mℓ |
| Lemon syrup | 200mℓ |
| Syrup | to 1000mℓ |

The above ingredients were mixed to produce a syrup which was packaged under a sterile condition in 6 oz. bottles. One teaspoon of this formulation was administrated to a 70kg male adult (age: 27), reducing inflammation and producing analgesia.

Use Example 4

Injectable compositions were prepared as follows:

| Ingredients | Amount |
|---|---|
| Composition 1: | |
| The Compound of Example 5 | 0.01% |
| Aqueous Acetic Acid(1.30%) | 95.45% |
| Dextrose | 4.54% |
| Composition 2: | |
| The Compound of Example 5 | 0.05% |
| Aqueous Sodium Acetate(1.18%) | 85.95% |
| Aqueous Acetic Acid(2.0%) | 10.00% |
| Benzyl alcohol | 4.04% |

The injection of 0.05mℓ of Composition 2 prior to oral surgery for a third molar extraction of a female adult (weight: 52kg; age: 29) successfully provided local anesthesia during the surgery.

Use Example 5

A composition for topical administration was prepared by combining the following ingredients:

| Ingredients | Amount |
|---|---|
| The Compound of Example 1 | 4g |
| Glycerol | 12mℓ |
| Purified water | 200mℓ |

The compound of Example 1 was dissolved in a solution containing the other ingredients. Application of 0.4mℓ of the resulting liquid to a $80cm^2$ portion of the forearm of a 60kg male adult produced local analgesia which lasted for two days. Little or no skin irritation was observed.

Tests of the activity

Physiological activities of the compounds prepared above were measured by employing the following methods.

1. Writhing Test

1) Animals tested

The KTC-ICR mice derived from Charles River Breeding Laboratory in the United States and provided by Experimental Animal Laboratory of Korea Research Institute of Chemical Technology were used as test animals. The mice subjected to the testing of those end products prepared in Examples 1 to 32 had a body weight of 20 to 25g; and those used for the testing of the compounds synthesized in Examples 33 to 46 had a weight ranging from 10 to 15g . They were tested after having been adjusted to the testing environment for a week. Food and water were given freely; and illumination was maintained on a 12-hour cycle.

2) Testing method

Experiments were performed in two ways: that is, the acetic acid induced writhing test and the phenyl-1,4-benzoquinone(PBQ) induced writhing test.

Solutions for the acetic acid induced writhing test were prepared by dissolving one of the end products prepared in Examples 1 to 35 in a saline solution containing 1% by weight of Tween 80 to have a concentration of 5mg/mℓ and diluting it serially with the saline solution. The test solutions were administered orally in a dose of 0.3mℓ per 30g of body weight, using the 5 ICR mice for each test. 60 minutes later, 0.9% acetic acid solution was administered intraperitoneally in a dose of 0.1mℓ per 30g of body weight. 3 minutes thereafter, the number of writhings generated during a period of 10 minutes due to the administration of acetic acid was measured. For comparison purpose, initially, saline solution alone was administered orally to the control group. 60 minutes thereafter, 0.9% acetic acid solution was administered intraperitoneally to the control group.

Alternatively, solutions for the PBQ induced writhing test were prepared by dissolving one of the products synthesized in Examples 33 to 46 in a mixture of Tween 80, alcohol and distilled water (1:5:94); and administered orally to the 5 ICR mice for each test in a dose of 0.3mℓ per 30g of body weight. 60 minutes later, 0.02% PBQ solution was administered intraperitoneally in a dose of 0.1mℓ per 30g of body weight of the test animals. 5 minutes thereafter, at the temperature of 40°C, the number of writhings occurred during a period of 5 minutes due to the PBQ solution administered was measured. For comparison, only the mixture of Tween 80, alcohol and distilled water was administered orally to the control group; and, after 60 minutes, the PBQ solution was administered intraperitoneally to the control group in the same manner as mentioned above.

3) Measurement of analgesic effect

The number of writhings suffered by the test group was compared with that of the control group; and the analgesic effect was measured in terms of the percentage of inhibition of writhing(I. W.).

$$\text{I. W. } (\%) = \frac{A - B}{A} \times 100$$

A : the number of writhings suffered by the control group,
B : the number of writhings suffered by the test group.

The amount of a test compound which is required in reducing the frequency of writhings to the 50% level of that generated by the control group, i.e., B=0.5A or I.W.=50%, is designated as $ED_{50}$: therefore, a lower value of $ED_{50}$ represents a higher analgesic effect of the tested compound. These $ED_{50}$ values for the test compounds are shown in the last column of Table 1.

The first column of Table 1 represents the number of each Example; n, Yp and X expressed in cols. 2 to 4 have the same meanings as defined in Formula(I); the data contained in cols. 5 to 7 describes the respective yield and melting point of the intermediates, i.e., compounds of the formulae(IV), (V) and (VI), employed in preparing the final product of formula(I), whose yield and melting point are stated in the eighth column.

Table 1

| Example | n | $Y_p$ | X | Formula(IV) | | Formula(V) | | Formula(VI) | | Formula(I) | | $ED_{50}$ (mg/Kg) |
|---|---|---|---|---|---|---|---|---|---|---|---|---|
| | | | | Yield | m.p. | Yield | m.p. | Yield | m.p. | Yield | m.p. | |
| 1 | 3 | 3,4-$Me_2$ | OMe | 90%, | -*1 | 63%, | 105°C | 97%, | 105°C | 94%, | 105°C | 0.08 |
| 2 | 3 | 3,4-$Me_2$ | H | 90%, | 100°C | 67%, | 97°C | 98%, | 105°C | 94%, | 92°C | 3.98 |
| 3 | 3 | 3,4-$Me_2$ | F | 88%, | - | 82%, | 97°C | 93%, | 83°C | 96%, | 69°C | 2.95 |
| 4 | 3 | 3,4-$Me_2$ | Cl | 82%, | 109°C | 90%, | 105°C | 89%, | 101°C | 93%, | 71°C | 4.78 |
| 5 | 3 | 3-Me | OMe | 91%, | - | 48%, | 116°C | 98%, | 87°C | 86%, | 88°C | 0.07 |
| 6 | 3 | 3-Me | H | 80%, | 88°C | 66%, | 95°C | 88%, | 79°C | 84%, | 88°C | 26.3 |
| 7 | 3 | 3-Me | F | 88%, | 78°C | 78%, | 86°C | 97%, | 55°C | 98%, | - | 2.3 |

Table 1(continued)

| Example | n | $Y_p$ | X | Formula(IV) | | Formula(V) | | Formula(VI) | | Formula(I) | | $ED_{50}$(mg/Kg) |
|---|---|---|---|---|---|---|---|---|---|---|---|---|
| | | | | Yield | m.p. | Yield | m.p. | Yield | m.p. | Yield | m.p. | |
| 8 | 3 | 3-Me | Cl | 96%, | 91°C | 75%, | 84°C | 98%, | 58°C | 97%, | - | 42.6 |
| 9 | 3 | 4-Me | OMe | 95%, | - | 82%, | 139°C | 95%, | 112°C | 92%, | 119°C | 1.73 |
| 10 | 3 | 4-Me | H | 88%, | 140°C | 87%, | 118°C | 98%, | 104°C | 54%, | 91°C | 7.9 |
| 11 | 3 | 4-Me | F | 92%, | 155°C | 72%, | 94°C | 80%, | 79°C | 76%, | 77°C | 0.01 |
| 12 | 3 | 4-Me | Cl | 90%, | 133°C | 91%, | 113°C | 99%, | 99°C | 45%, | 78°C | 4.7 |
| 13 | 3 | 3,5-$Me_2$ | OMe | 95%, | - | 60%, | 109°C | 77%, | 90°C | 75%, | 90°C | 4.6 |
| 14 | 3 | 3,5-$Me_2$ | H | 63%, | 75°C | 70%, | 114°C | 80%, | 83°C | 82%, | 99°C | 3.16 |
| 15 | 3 | 3,5-$Me_2$ | F | 90%, | 105°C | 95%, | 91°C | 94%, | 65°C | 75%, | 98°C | 0.02 |
| 16 | 3 | 3,5-$Me_2$ | Cl | 92%, | - | 70%, | 89°C | 84%, | 68°C | 94%, | 99°C | 123 |
| 17 | 2 | 3,5-$Me_2$ | OMe | 78%, | - | 60%, | 130°C | - | 109°C | 87%, | 118°C | 0.77 |

Table 1(continued)

| Example | n | Yp | X | Formula(IV) | | Formula(V) | | Formula(VI) | | Formula(I) | | $ED_{50}$(mg/Kg) |
|---|---|---|---|---|---|---|---|---|---|---|---|---|
| | | | | Yield | m.p. | Yield | m.p. | Yield | m.p. | Yield | m.p. | |
| 18 | 3 | 4-Cl | OMe | 98%, | - | 64%, | 118°C | 93%, | 95°C | 92%, | 121°C | 0.40 |
| 19 | 3 | 4-Cl | H | 90%, | 81°C | 98%, | 108°C | 93%, | 97°C | 95%, | 92°C | 0.03 |
| 20 | 3 | 4-Cl | F | 75%, | - | 95%, | 74°C | 82%, | 63°C | 41%, | 91°C | 0.07 |
| 21 | 3 | 4-Cl | Cl | 77%, | 136°C | 83%, | 95°C | 92%, | 77°C | 94%, | 77°C | 0.04 |
| 22 | 3 | 4-F | OMe | - | - | - | - | 80%, | - | - | - | - |
| 23 | 3 | 2,4-$Cl_2$ | OMe | 89%, | - | - | - | - | - | - | - | - |
| 24 | 3 | 3,4-$Cl_2$ | OMe | 84%, | - | 68%, | 102°C | 80%, | 108°C | 75%, | 114°C | 0.66 |
| 25 | 3 | 3,4-$(CH_2O_2)_2$ | OMe | 84%, | - | 67%, | 125°C | 94%, | 112°C | 92%, | 106°C | 0.8 |
| 26 | 3 | 3-$CF_3$ | OMe | 98%, | - | 62%, | 104°C | 95%, | 92°C | 91%, | 90°C | 1.54 |
| 27 | 3 | 3-OMe | OMe | - | - | - | - | - | - | - | - | 1.25 |

Table 1(continued)

| Example | n | $Y_p$ | X | Formula(IV) | | Formula(V) | | Formula(VI) | | Formula(I) | | $ED_{50}$(mg/Kg) |
|---|---|---|---|---|---|---|---|---|---|---|---|---|
| | | | | Yield | m.p. | Yield | m.p. | Yield | m.p. | Yield | m.p. | |
| 28 | 3 | 3-$OCH_2Ph$ | OMe | 65%, | - | - | - | - | - | - | - | 28.8 |
| 29 | 3 | 3,4-$(OMe)_2$ | OMe | 93%, | - | 61%, | 126°C | 93%, | 86°C | 91%, | 126°C | - |
| 30 | 4 | 3-Me | OMe | 91%, | - | 65%, | 92°C | 98%, | 63°C | 90%, | 107°C | 1.54 |
| 31 | 4 | 3-Me | H | 84%, | 85°C | 84%, | 92°C | 81%, | 83°C | 89%, | 113°C | 3.3 |
| 32 | 4 | 3,4-$Me_2$ | F | - | - | - | - | - | - | - | - | 1.44 |
| 33 | 3 | 3,4-$Me_2$ | OH | 87%, | 66°C | 85%, | 75°C | 84%, | 106°C | 87%, | 147°C | 0.49 |
| 34 | 3 | 3-Me | OH | 89%, | - | 87%, | 93°C | 97%, | 96°C | 92%, | dec. | - |
| 35 | 3 | 4-OMe | OH | 66%, | - | 85%, | 118°C | 57%, | 112°C | 61%, | 125°C | - |
| 36 | 3 | 3,4-$(CH_2O_2)_2$ | OH | 47%, | - | - | - | - | 115°C | - | - | - |
| 37 | 3 | 3,4-$Me_2$ | $NH_2$ | 90%, | 103°C | 94%, | 93°C | 97%, | 89°C | 94%, | 98°C | 22.7 |

Table 1(continued)

| Example | n | Yp | X | Formula(IV) | | Formula(V) | | Formula(VI) | | Formula(I) | | $ED_{50}$(mg/Kg) |
|---|---|---|---|---|---|---|---|---|---|---|---|---|
| | | | | Yield | m.p. | Yield | m.p. | Yield | m.p. | Yield | m.p. | |
| 38 | 3 | 3-Me | $NH_2$ | - | 97°C | 88%, | 58°C | 94%, | 50°C | 97%, | 74°C | 22.1 |
| 39 | 3 | 4-Cl | $NH_2$ | 82%, | 90°C | 81%, | 58°C | 99%, | 115°C | 99%, | 249°C | 77.6 |
| 40 | 3 | 4-F | $NH_2$ | 85%, | 132°C | 84%, | 78°C | 99%, | 101°C | 99%, | 238°C | 37.8 |
| 41 | 3 | 3,4-$Cl_2$ | $NH_2$ | 94%, | 115°C | 99%, | 55°C | 99%, | 89°C | 99%, | 240°C | 7.18 |
| 42 | 3 | 3-$CF_3$ | $NH_2$ | 83%, | 94°C | 87%, | 112°C | 94%, | 75°C | 93%, | 190°C | - |
| 43 | 3 | 3,4-$Me_2$ | $NO_2$ | - | | - | | - | | 82%, | 98°C | 22.5 |

Table 1(continued)

| Example | n | $Y_p$ | X | Formula(IV) | | Formula(V) | | Formula(VI) | | Formula(I) | | $ED_{50}$(mg/Kg) |
|---|---|---|---|---|---|---|---|---|---|---|---|---|
| | | | | Yield | m.p | Yield | m.p | Yield | m.p | Yield | m.p | |
| **44** | 3 | 4-F | $NO_2$ | - | | - | | - | | - | - | - |
| **45** | 3 | 3,4-$Cl_2$ | $NO_2$ | - | | - | | - | | 40%, | 83°C | - |
| **46** | 3 | 3-$CF_3$ | $NO_2$ | - | | - | | - | | 62%, | 60°C | - |
| NE-21610*2 | | | | | | | | | | | | >300 |

*1 "-" signs represent data not available.

*2 N-(4-(2-aminoethoxy)-3-methoxybenzyl))oleamide, The Procter & Gamble Company (covered in USP 5,045,565)

2. Behavior Analysis

In order to monitor a harmful side-effect or toxicity of the compounds having the formula(I), various behavioral changes in the test animals were observed. After the test and the control solutions were administered to the animals, such symptoms as sedation, ptosis, dyspnoea, vasodilation, convulsion, salivation and urination were observed and the level of such changes was represented by a numbering system: that is, the normal value of the last three behaviors(i.e., urination, convulsion and salivation) is 0; and that of the others(i.e.; sedation, ptosis, dyspnoea and vasodilation) is 4. The higher the number is, the greater the side effects are. The test results for some of the compounds are shown in Table 2.

Table 2

| Compounds | | Behavior | | | | | | |
|---|---|---|---|---|---|---|---|---|
| Example | dose | Sedation | Ptosis | Dyspnoea | Vasodilation | Convulsion | Salivation | Urination |
| 33 | 2.0mg/Kg | 7 | 6 | 5 | 4 | 0 | 0 | 0 |
| | 0.6mg/Kg | 5 | 4 | 4 | 4 | 0 | 0 | 0 |
| | 0.2mg/Kg | 4 | 4 | 4 | 4 | 0 | 0 | 0 |
| 37 | 60mg/Kg | 6 | 6 | 4 | 4 | 0 | 0 | 0 |
| | 20mg/Kg | 4 | 5 | 4 | 4 | 0 | 0 | 0 |
| | 6.7mg/Kg | 4 | 4 | 4 | 4 | 0 | 0 | 0 |
| 38 | 60mg/Kg | 6 | 7 | 7 | 4 | 0 | 0 | 0 |
| | 20mg/Kg | 4 | 5 | 4 | 4 | 0 | 0 | 0 |
| | 6.7mg/Kg | 4 | 4 | 4 | 4 | 0 | 0 | 0 |

Table 2(continued)

| Compounds | | Behavior | | | | | | |
|---|---|---|---|---|---|---|---|---|
| Example | dose | Sedation | Ptosis | Dyspnoea | Vasodilation | Convulsion | Salivation | Urination |
| 39 | 50mg/Kg | 4 | 4 | 4 | 4 | 0 | 0 | 0 |
| | 25mg/Kg | 4 | 4 | 4 | 4 | 0 | 0 | 0 |
| | 10mg/Kg | 4 | 4 | 4 | 4 | 0 | 0 | 0 |
| 40 | 50mg/Kg | 4 | 4 | 4 | 4 | 0 | 0 | 0 |
| | 25mg/Kg | 4 | 4 | 4 | 4 | 0 | 0 | 0 |
| | 10mg/Kg | 4 | 4 | 4 | 4 | 0 | 0 | 0 |
| 41 | 100mg/Kg | 6 | 5 | 5 | 4 | 0 | 0 | 0 |
| | 50mg/Kg | 5 | 4 | 4 | 4 | 0 | 0 | 0 |
| | 25mg/Kg | 4 | 4 | 4 | 4 | 0 | 0 | 0 |
| 42 | 100mg/Kg | 7 | 7 | 4 | 4 | 0 | 0 | 0 |
| | 33.3mg/Kg | 4 | 4 | 4 | 4 | 0 | 0 | 0 |
| | 11.1mg/Kg | 4 | 4 | 4 | 4 | 0 | 0 | 0 |
| NE-21610 | 100mg/Kg | 7 | 7 | 6 | 4 | 2 | 0 | 0 |
| | 50mg/Kg | 5 | 5 | 4 | 4 | 0 | 0 | 0 |
| | 25mg/Kg | 4 | 4 | 4 | 4 | 0 | 0 | 0 |
| | 10mg/Kg | 4 | 4 | 4 | 4 | 0 | 0 | 0 |

3. Randal-Selitto Test

Randall-Selitto Test was carried out by following the method described in Arch. Int. Pharmacodyn. 11, 409(1957).

Male albino rats(120-170g) of the Charles River Sprague-Dawley strain were used. Inflammation was produced by the injection of 0.1mℓ of a 20% suspension of Brewer's yeast into the plantar surface of the rat's hind foot. Thresholds were determined using a modified apparatus described in Winter and Flataker(J. Pharm. Exp. Ther. 148, 373(1965)).

The pain threshold was measured as the pressure in mmHg required to induce the desired response(a sharp audible squeak and/or struggle) when the pressure was applied to the foot. Air pressure from an air line was admitted through a needle valve to a 20mℓ glass syringe and to a pressure gauge which was connected by a T-tube. The syringe was mounted with the plunger downward to which was connected a short bullet-shaped Teflon peg. The pressure was applied to the foot of the rat at the rate of 10 mmHg per second. Drug was given 2 hours after the yeast injection. Two hours after the drug administration, threshold response was determined. The results were compared with the results obtained from the yeast-treated, saline control group, as shown in Table 3.

The analgesic activity was determined in terms of the percentage of inhibition of response:

$$\text{Inhibition(\%)} = \frac{\text{Threshold of the treated group - Threshold of the control group}}{\text{Threshold of the control group}} \times 100$$

The compound of Example 1, administered two hours after yeast injection and one hour before the test at a dose of 5mg/kg perorally, caused an inhibition of yeast induced hyperalgesia.

Table 3

| Compound | Dose(mg/Kg) | Number of rats | Inhibition(%) |
|---|---|---|---|
| Aspirin[1] | 100(s. c.) | 10 | 80.1 |
| Ketoprofen[2] | 10(p. o.) | 8 | 62.1 |
| Morphine[3] | 3(s. c.) | 8 | 391.7 |
| Example 1 | 5(p. o.) | 5 | 247.4 |
| Example 1-HCl | 1.5(s. c.) | 8 | 248.3 |

1. Bayer, US-A-3,235,583
2. Rhone-Poulenc, US-A-3,641,127
3. US-A-2,740,787

4. Tail-flick Test

The tail flick assay of D'Amour and Smith(J. Pharm. Exp. Ther. 72, 74(1941)) was modified for use with mice. Radiant heat was applied using a beam of high-intensity light focused on a tail spot. The response time, defined as the interval between the onset of the stimulus and the tail flick, was measured electronically(to the nearest 0.1 second). The beam intensity was set at a level giving a mean control reaction time of 3.8±0.4 seconds. Animals that did not flick their tails within 15 seconds were removed and assigned a 15-second response latency.

The inhibition rates(analgesic effects) of the compound of Example 1 and standard compounds are shown in Table 4. The percentage of inhibition was determined by the following equation:

$$\text{Inhibition(\%)} = \frac{\text{Reaction time of the treated group - Reaction time of the control group}}{\text{Reaction time of the control group}} \times 100$$

Morphine used as reference was active in this test; but nonsteroidal anti-inflammatory drugs were ineffective. The results show that the compound of Example 1 is more effective than morphine and suggest that the Example 1 compound behaves as a central analgesic.

Table 4

| Compound | Dose(mg/Kg) | Inhibition(%) |
|---|---|---|
| Aspirin(p. o.) | 100 | 0 |
| Piroxicam(p. o.)[1] | 100 | 0 |
| Capsaicin | 25 | 90 |
| Morphine-HCl(s. c.) | 2.0 | 57 |
| | 5.0 | 100 |
| NE-21610(p. o.)[2] | 200 | 50 |
| Example 1(s. c.) | 0.5 | 50 |
| | 1.0 | 70 |
| Example 1(p. o.) | 1.25 | 10 |
| | 2.5 | 60 |
| | 5.0 | 80 |
| | 7.5 | 90 |
| Example 1-l-tartarate (p. o.) | 1.25 | 50 |
| | 2.5 | 90 |
| Example 40(p. o.) | 100 | 50 |
| Example 40-HCl(p. o.) | 50 | 67 |
| Example 42(p. o.) | 100 | 40 |
| Example 50(p. o.) | 200 | 38 |

1. Pfizer, US-A-3,591,584
2. P & G, US-A-5,045,565

5. Hot-plate Test

Mice were placed on an aluminum plate maintained at 55± 0.5°C by a thermo-regulator(Harvard). A glass cylinder, 15cm in height and 15cm in diameter, served to confine the mice to the heated plate. Blowing of the fore paws was used as the end-point for determination of response latency(measured to the neatest 0.1 second). Animals which failed to react within 30 seconds were removed and assigned a 30-second response latency.

The inhibition rates of the compound of Example 1(p. o.) and morphine(s. c.) in the hot plate test were determined by the same equation as used in Tail-flick Test, which are shown in Table 5. This result shows that the compound of Example 1(p. o.) is as effective as morphine(s. c.) and also suggests that the compound of Example 1 behaves as a central analgesic.

Table 5

| Compound | Dose(mg/Kg) | Inhibition(%) |
|---|---|---|
| Morphine-HCl(s. c.) | 2.0 | 27.5 |
| | 5.0 | 49.0 |
| | 10.0 | 98.0 |
| Example 1(p. o.) | 2.5 | 28.6 |
| | 5.0 | 48.9 |
| | 10.0 | 97.0 |

6. Tail-pinch test in rats with hyperalgesia induced by Freund's adjuvant

Rats(Sprague-Dawley) weighing 120g to 170g were used. Desiccated Mycobacterium butyricum(Difco Laboratories, Detroit, MI) was ground in a mortar, suspended in liquid paraffin, sterilized in an autoclave, and injected(0.5mg in 0.1mℓ, s.c.) in the distal region of the tail through a 1-inch 21-gauge needle.

Animals so treated exhibited hypersensitivity to the pressure placed on the tail within a few hours of the injection and were used for analgesic testing 18 to 24 hours after injection. The hypersensitivity of the tail was examined as follows: the animal was held comfortably in one hand and gentle pressure was applied with the fingers of the opposite hand to the injected area. This gentle squeeze or "tail pinch" elicited a "squeak" from the animal. Five such stimuli were given at 4-second intervals. If the animal emitted no more than one squeak in five trials, it was recorded as having analgesia and given a rating of 1. If there was more than one squeak, the rating was given the value of 0.

The analgesic activity was determined by the following equation:

$$\text{Analgesic activity} = \frac{\text{Total rating}}{\text{Tested aminal number}} \times 100$$

The compound of Example 1, administered two hours before tail-pinch testing perorally, caused a dose related inhibition of adjuvant induced hyperalgesia, as shown in Table 6.

Table 6

| Compound | Dose(mg/Kg) | Number of rats | Analgesic activity(%) |
|---|---|---|---|
| Naproxen[1] | 5 | 7 | 28.6 |
| Example 1 | 5 | 7 | 45.9 |
| | 10 | 6 | 66.7 |

1. Syntex, US-A-3,637,767

7. Anti-inflammatory Test

Rats(Sprague-Dawley, female) weighing 100 to 120g were used. Twenty minutes after the test drug was administered(s. c.), carrageenin was injected(0.1mℓ of 1% solution, s. c.) in the plantar surface of the right hind paw. The volume of the edema was measured with a volumeter(Rehma Volumeter 2060) 3 hours later.

The percentage of inhibition was determined by the following equation:

$$\text{Inhibition(\%)} = \frac{\text{Volume of the carrageenin treated foot - Volume of the control foot}}{\text{Volume of the contol foot}} \times 100$$

The amount of a test compound which is required in obtaining 50% inhibition is designated as $ED_{50}$.

The inhibitory effects of Example 1 and 5 were comparable to that of Ketoprofen, but significantly superior to that of Aspirin or Naproxen, as shown in table 7.

Table 7

| Compound | $ED_{50}$(mg/Kg) | Compound | $ED_{50}$(mg/Kg) |
|---|---|---|---|
| Ketoprofen | 7.2 | Naproxen | 43.0 |
| Aspirin | 109 | Example 5 | 16.9 |
| Example 1 | 4.0 | | |

8. Local Anti-inflammatory Test

Rats(Sprague-Dawley, female) weighing 100 to 120g were used. 20 minutes after the test drug was administered

transdermally (another application 7 hours later for double dose experiment), carrageenin was injected(0.1mℓ of 1% solution, s. c.) into the plantar surface of the right hind paw. The volume of the edema was measured with a volumeter either 1 hour later for signle dose experiment or 24 hours later for double dose experiment; and the percentage of inhibition was measured by the same equation as used in Anti-inflammatory Test.

The inhibitory effect of the compound of Example 1 was slightly better than that of Naproxen, as shown in Table 8.

Table 8

| Compound | Single Dose(1 hr) | Double Dose(24 hr) |
|---|---|---|
| Narproxen | 54% | 25% |
| Example 1 | 66% | 32% |

Test of toxcity

In addition, the acute toxicity test for the compounds of the present invention was carried out at $LD_{50}$ by per os administering the test compounds in varied amounts in a stepwise manner into 5 ICR male mice and 5 ICR female mice(5 weeks old), which were observed for 14 days. The $LD_{50}$ values of the test compounds are as shown in Table 9.

Table 9

| Compound | $LD_{50}$(male), mg/kg | $LD_{50}$(female), mg/kg |
|---|---|---|
| Example 1 | 224 | 364 |
| Example 5 | 125 | 125 |
| Example 11 | 500 | 322 |
| Example 15 | >2,000 | >2,000 |

Test of mutagenecity

The mutagenecity test for the compound of Example 1 was carried out using Salmonella typhimurium TA98 and TA 100 which required histidine for growth, according to the method of Maron and Ames(Mutation Research, 1983, 113, 173-275). The numbers of mutated colonies were not increased at concentrations of 3.2, 16, 80 and 400µg/plate for the test compound.

## Claims

1. Phenylacetamide compounds and pharmaceutically accceptable salts thereof, of formula (I)

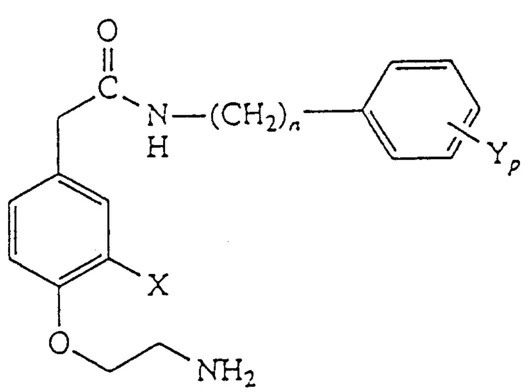

(I)

wherein:

X is a hydrogen, halogen, hydroxy, nitro, amino, $R^1$, $NR^1R^2$, $NHR^1$ or $OR^1$ wherein $R^1$ and $R^2$ are an optionally substituted $C_{1-8}$ alkyl, cycloalkyl or benzyl group, respectively,

Y, which may be the same or different when p is greater than 1, is a halogen, methylenedioxy, hydroxy, trifluoromethyl, $R^3$ or $OR^3$ wherein $R^3$ is an optionally substituted $C_{1-8}$ alkyl or benzyl group;

n is an integer from 1 to 6; and

p is an integer from 1 to 5.

**2.** The compound of claim 1 wherein X is a hydrogen, halogen, hydroxy, nitro, amino or $OR^1$ wherein $R^1$ is an optionally substituted $C_{1-8}$ alkyl, cycloalkyl or benzyl group; Y, which may be the same or different when p is greater than 1, is a halogen, trifluoromethyl or $R^3$ wherein $R^3$ is an optionally substituted $C_{1-8}$ alkyl or benzyl group; n is an integer from 2 to 5; and p is an integer from 1 to 3.

**3.** The compound of claim 1 wherein X is a halogen, hydroxy, nitro, amino or $OR^1$; Y, which may be the same or different when p is 2, is a halogen, trifluoromethyl or $R^3$, wherein $R^1$ and $R^3$ are an optionally substituted $C_{1-5}$ alkyl group, respectively; n is 3 or 4; and p is 1 or 2.

**4.** The compound of claim 3 wherein X is a methoxy group.

**5.** The compound of claim 3 wherein X is a hydroxy group.

**6.** The compound of claim 3 wherein X is a nitro or amino group.

**7.** Pharmaceutically acceptable salts of the compounds of claim 1 wherein the salts are selected from the group consisting of salts obtained by reacting with inorganic acids including hydrochloric acid, hydrogen bromide, sulfuric acid, sodium hydrogen sulfate and carbonic acid; and with organic acids including formic, acetic, oxalic, benzoic, citric, tartaric, gluconic, gentisic, fumaric and lactobionic acids.

**8.** A process for preparing a compound having formula (I) of claim 1 which comprises the steps of:

(i) reacting an amine compound of formula II with a parahydroxyphenylacetic acid of formula (III) in the presence of a powdered 0.3 nm, 0.4 nm or 0.5 nm (3Å, 4Å or 5Å) molecular sieve at a temperature ranging from 130°C to 160°C for a period ranging from 2 to 5 hours to produce a compound of formula (IV);
(ii) reacting the compound of formula (IV) with 1,2-dibromoethane to produce a compound of formula (V);
(iii) reacting the compound of formula (V) with sodium azide ($NaN_3$) to produce a compound of formula (VI); and, thereafter
(iv) converting the compound of formula (VI) to the compound of formula (I) by reducing the compound of formula (VI) in the presence of a catalyst under an elevated hydrogen pressure:

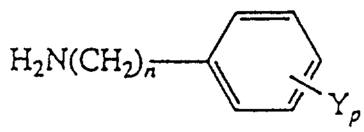 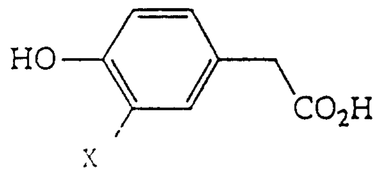

(II) (III)

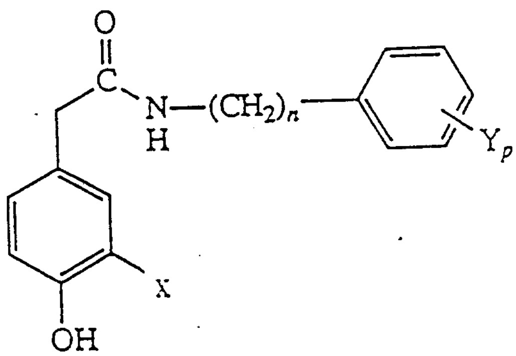 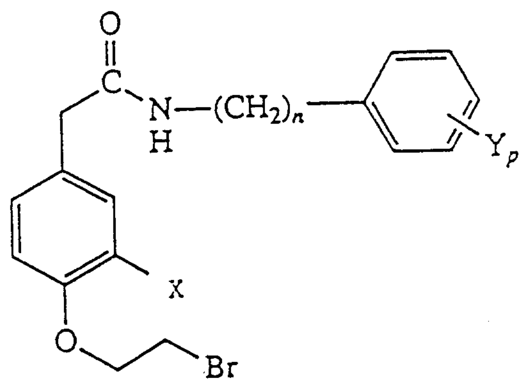

(IV) (V)

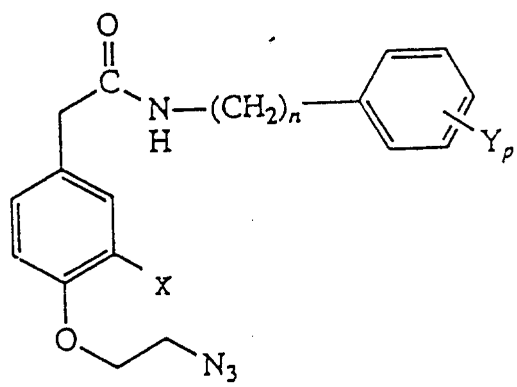 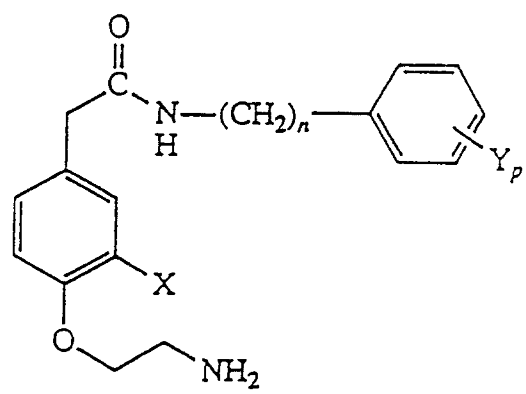

(VI) (I)

wherein:

X, Y, n and p have the same meanings as defined in claim 1.

**9.** A pharmaceutical composition comprising as an active ingredient thereof a phenylacetamide compound of formula (I) as defined in claim 1.

**Patentansprüche**

1. Phenylacetamidverbindungen der Formel (I) und pharmazeutisch verträgliche Salze davon

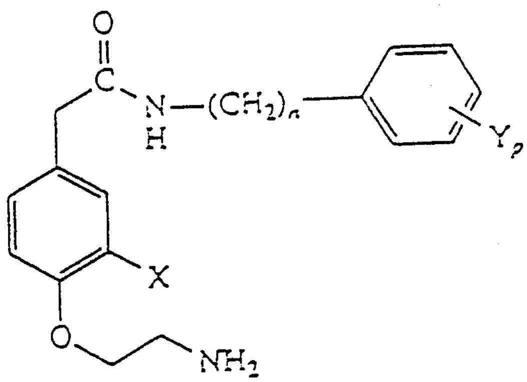

(I)

worin

X ein Wasserstoff, Halogen, Hydroxy, Nitro, Amino, $R^1$, $NR^1R^2$, $NHR^1$ oder $OR^1$ ist, worin $R^1$ und $R^2$ eine gegebenenfalls substituierte $C_{1-8}$-Alkyl-, Cycloalkyl- bzw. Benzylgruppe sind,
Y, das gleich oder verschieden sein kann wenn p größer als 1 ist, ein Halogen, Methylendioxy, Hydroxy, Trifluormethyl, $R^3$ oder $OR^3$ ist, worin $R^3$ eine gegebenenfalls substituierte $C_{1-8}$-Alkyl- oder Benzylgruppe ist;
n eine ganze Zahl von 1 bis 6 ist; und
p eine ganze Zahl von 1 bis 5 ist.

2. Verbindung nach Anspruch 1, worin X ein Wasserstoff, Halogen, Hydroxy, Nitro, Amino oder $OR^1$ ist, worin $R^1$ eine gegebenenfalls substituierte $C_{1-8}$-Alkyl-, Cycloalkyl- oder Benzylgruppe ist; Y, das gleich oder verschieden sein kann wenn p größer als 1 ist, ein Halogen, Trifluormethyl oder $R^3$ ist, worin $R^3$ eine gegebenenfalls substituierte $C_{1-8}$-Alkyl- oder Benzylgruppe ist; n eine ganze Zahl von 2 bis 5 ist, und p eine ganze Zahl von 1 bis 3 ist.

3. Verbindung nach Anspruch 1, worin X ein Halogen, Hydroxy, Nitro, Amino oder $OR^1$ ist; Y, das gleich oder verschieden sein kann wenn p 2 ist, ein Halogen, Trifluormethyl oder $R^3$ ist, worin $R^1$ und $R^3$ jeweils eine gegebenenfalls substituierte $C_{1-5}$-Alkylgruppe sind; n 3 oder 4 ist und p 1 oder 2 ist.

4. Verbindung nach Anspruch 3, worin X eine Methoxygruppe ist.

5. Verbindung nach Anspruch 3, worin X eine Hydroxygruppe ist.

6. Verbindung nach Anspruch 3, worin X eine Nitro- oder Aminogruppe ist.

7. Pharmazeutisch verträgliche Salze der Verbindungen nach Anspruch 1, worin die Salze aus der Gruppe ausgewählt werden, die aus Salzen besteht, die durch Umsetzen mit anorganischen Säuren, einschließlich von Salzsäure, Bromwasserstoff, Schwefelsäure, Natriumhydrogensulfat und Kohlensäure, und mit organischen Säuren, einschließlich Ameisen-, Essig-, Oxal-, Benzoe-, Zitronen-, Wein-, Glucon-, Gentisin-, Fumar- und Lactobionsäure, erhalten werden.

8. Verfahren zur Herstellung einer Verbindung der Formel (I) nach Anspruch 1, das die folgenden Schritte umfaßt:

(i) Umsetzen einer Aminverbindung der Formel II mit einer para-Hydroxyphenylessigsäure der Formel (III) in Gegenwart eines pulverförmigen 0,3 nm, 0,4 nm oder 0,5 nm (3 Å, 4 Å oder 5 Å) Molekularsiebs bei einer Temperatur im Bereich von 130 bis 160 °C, während eines Zeitraums im Bereich von 2 bis 5 Stunden, wodurch eine Verbindung der Formel (IV) hergestellt wird;
(ii) Umsetzen der Verbindung der Formel (IV) mit 1,2-Dibromethan zur Herstellung einer Verbindung der Formel (V);
(iii) Umsetzen der Verbindung der Formel (V) mit Natriumazid ($NaN_3$) zur Herstellung einer Verbindung der Formel (VI); und danach

(iv) Umwandeln der Verbindung der Formel (VI) in die Verbindung der Formel (I) durch Reduzieren der Verbindung der Formel (VI) in Gegenwart eines Katalysators unter einem erhöhten Wasserstoffdruck:

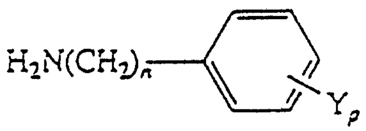

(II)

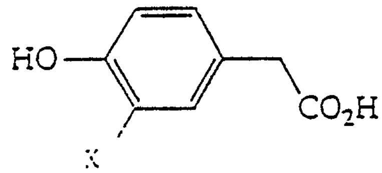

(III)

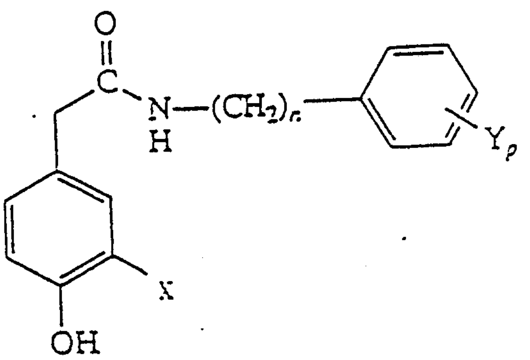

(IV)

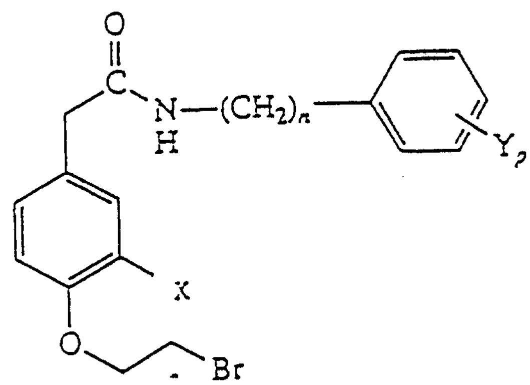

(V)

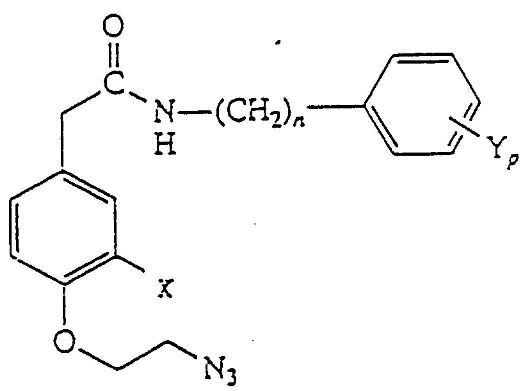

(VI)

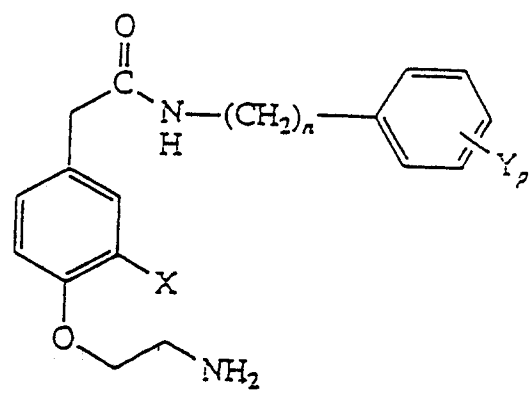

(I)

worin

X, Y, n und p die gleichen Bedeutungen aufweisen, wie in Anspruch 1 definiert.

**9.** Pharmazeutische Zusammensetzung, die als aktiven Bestandteil eine Phenylacetamidverbindung der Formel (I) nach Anspruch 1 enthält.

**Revendications**

1. Dérivés de phénylacétamide et leurs sels pharmaceutiquement acceptables, de formule (I)

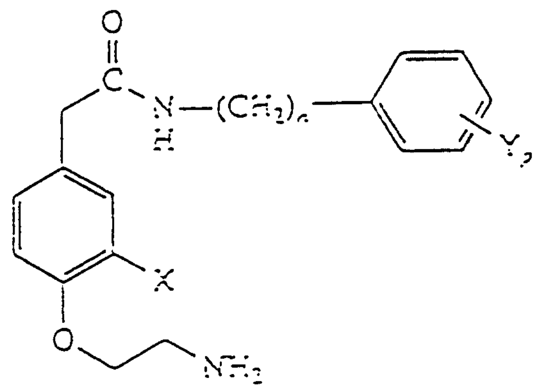

(I)

dans laquelle :

X représente un atome d'hydrogène ou d'halogène ou un groupe hydroxy, nitro, amino, $R^1$, $NR^1R^2$, $NHR^1$ ou $OR^1$, où $R^1$ et $R^2$ représentent respectivement un groupe benzyle, cycloalkyle en alkyle en $C_{1-8}$ éventuellement substitué,
Y, qui peuvent être identiques ou différents lorsque p est supérieur à 1, représentent un atome d'halogène ou un groupe méthylènedioxy, hydroxy, trifluorométhyle, $R^3$ ou $OR^3$ où $R^3$ est un groupe benzyle ou alkyle en $C_{1-8}$ éventuellement substitué ;
n représente un nombre entier valant de 1 à 6, et
p représente un nombre entier valant de 1 à 5.

2. Composé selon la revendication 1, dans lequel X représente un atome d'hydrogène ou d'halogène ou un groupe hydroxy, nitro, amino ou $OR^1$ où $R^1$ représente un groupe benzyle, cycloalkyle ou alkyle en $C_{1-8}$ éventuellement substitué ; Y, qui peuvent être identiques ou différents lorsque p est supérieur à 1, représente un atome d'halogène ou un groupe trifluorométhyle ou $R^3$, où $R^3$ est un groupe benzyle ou alkyle en $C_{1-8}$ éventuellement substitué ; n représente un nombre entier valant de 2 à 5 et p représente un nombre entier valant de 1 à 3.

3. Composé selon la revendication 1, dans lequel X représente un atome d'halogène ou un groupe hydroxy, nitro, amino ou $OR^1$; Y, qui peuvent être identiques ou différents lorsque p vaut 2, représentent un atome d'halogène ou un groupe trifluorométhyle ou $R^3$, $R^1$ et $R^3$ étant respectivement un groupe alkyle en $C_{1-5}$ éventuellement substitué; n vaut 3 ou 4 ; et p vaut 1 ou 2.

4. Composé selon la revendication 3, dans lequel X représente un groupe méthoxy.

5. Composé selon la revendication 3, dans lequel X représente un groupe hydroxy.

6. Composé selon la revendication 3, dans lequel X représente un groupe nitro ou amino.

7. Sels pharmaceutiquement acceptables des composés selon la revendication 1, dans lesquels les sels sont choisis dans le groupe comprenant les sels obtenus par réaction avec des acides minéraux englobant l'acide chlorhydrique, le bromure d'hydrogène, l'acide sulfurique, l'hydrogénosulfate de sodium et l'acide carbonique ; et avec des acides organiques englobant les acides formique, acétique, oxalique, benzoïque, citrique, tartrique, gluconique, gentisique, fumarique et lactobionique.

8. Procédé pour la préparation d'un composé répondant à la formule (I) selon la revendication 1, qui comprend les étapes de :

(i) réaction d'un dérivé d'amine de formule (II) avec un acide para-hydroxyphénylacétique de formule (III) en

présence d'un tamis moléculaire pulvérulent de 0,3 nm, 0,4 nm ou 0,5 nm (3 Å, 4 Å ou 5 Å) à une température se situant de 130°C à 160°C pendant une période allant de 2 à 5 heures pour produire un composé de formule (IV) ;

(ii) réaction du composé de formule (IV) avec le 1,2-dibromoéthane pour produire le composé de formule (V) ;

(iii) réaction du composé de formule (V) avec l'azoture de sodium ($NaN_3$) pour produire un composé de formule (VI) ; et ensuite

(iv) conversion du composé (VI) en le composé de composé de formule (I) en réduisant le composé de formule (VI) en présence d'un catalyseur sous une pression d'hydrogène élevée :

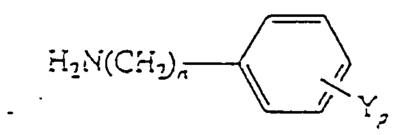

(II)

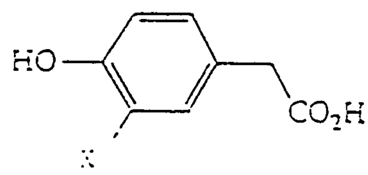

(III)

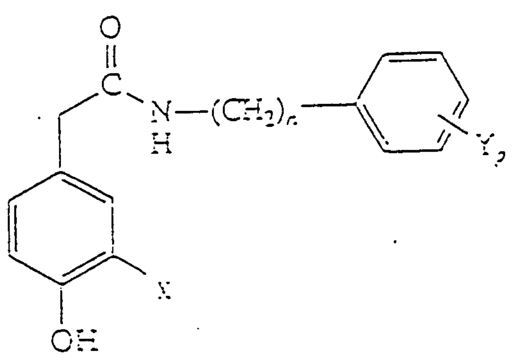

(IV)

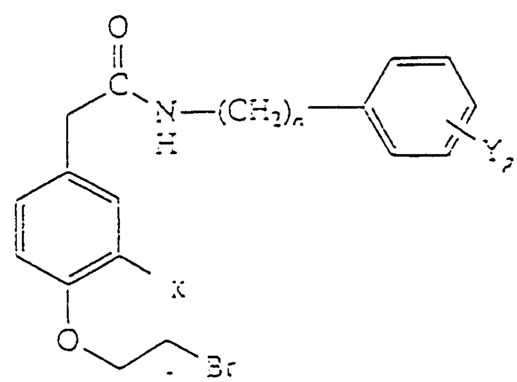

(V)

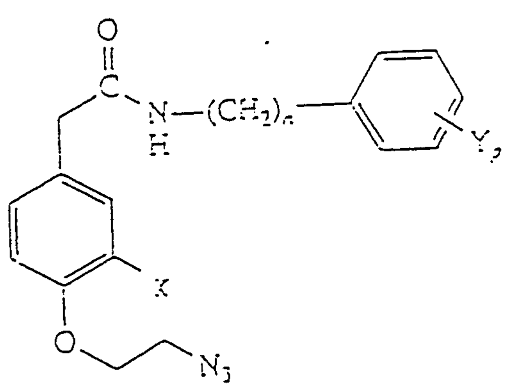

(VI)

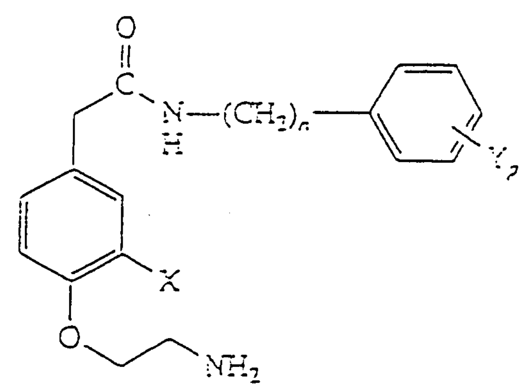

(I)

formules dans lesquelles X, Y, n et p ont les mêmes significations que données dans la revendication 1.

9. Composition pharmaceutique comprenant, à titre d'un de ses ingrédients actifs, un dérivé de phénylacétamide de formule (I) tel que défini dans la revendication 1.